# EUROPÄISCHE PATENTSCHRIFT

(11) **EP 2 238 967 B1**
(45) Veröffentlichungstag und Bekanntmachung des Hinweises auf die Patenterteilung: **02.05.2018**
(21) Anmeldenummer: 10156741.0
(22) Anmeldetag: 17.03.2010
(51) Int. Cl.: A61K 8/35, A61K 8/49, A61K 8/66, A61Q 19/08

(54) **Hautbehandlungsmittel gegen Hautalterung II**
Skin treatment fighting skin aging II
Moyen de traitement de la peau contre le vieillissement II

(30) Priorität: 06.04.2009 DE 102009002226
(43) Veröffentlichungstag der Anmeldung: 13.10.2010
(73) Patentinhaber: Henkel AG & Co. KGaA, 40589 Düsseldorf (DE)
(72) Erfinder: Janßen, Frank, 41470, Neuss (DE); Dickhof, Susanne, 41748, Viersen (DE); Holtkötter, Olaf, 50354, Hürth (DE); Emond, Eliane, 40470, Düsseldorf (DE)

(56) Entgegenhaltungen:
- EP-A1- 1 284 133
- EP-A1- 1 952 845
- EP-A2- 1 430 882
- WO-A1-2007/128723
- WO-A1-2008/111796
- WO-A2-2009/023416
- DE-A1-102008 025 089
- FR-A1- 2 768 927
- JP-A- H04 134 043
- "Lipochroman-6", , 24 February 2005 (2005-02-24), XP055268666, Retrieved from the Internet: URL:https://www.lotioncrafter.com/referenc e/tech_data_lipochroman_6.pdf [retrieved on 2016-04-26]

## Beschreibung

Die Erfindung betrifft topische kosmetische oder dermatologische Zusammensetzungen zur Hautbehandlung, die der Hautalterung und deren Anzeichen entgegenwirken.

Mittel und Behandlungsmethoden zur Behandlung reifer oder intrinsisch oder extrinsisch gealterter oder lichtgeschädigter Haut, insbesondere zur Antifaltenbehandlung, sind eine bedeutende kosmetische Herausforderung.

Der Alterungsprozess ist gekennzeichnet durch einen fortschreitenden Übergang der Hautzellen aus einem proliferativen Status in einen ruhenden, seneszenten Status. Dieser Übergang entspricht der Alterung auf zellulärer Ebene, auf den sich ein Großteil der makroskopischen Alterungseffekte zurückführen lässt. Die Zellzahl im Gewebe verringert sich und kann mangels proliferierender Zellen nicht mehr aufgefüllt werden. Dadurch können keine Reparaturen des umliegenden Gewebes durchgeführt werden, Defekte reichern sich an, wodurch die Haut atrophisch wird und erschlafft. Verstärkt wird dieser Effekt unter anderem auch durch UV-Licht, das die Haut ebenfalls belastet und deren Alterung und Erschlaffung beschleunigt.

Der vorliegenden Erfindung lag die Aufgabe zugrunde, Zubereitungen zur Behandlung und deutlichen Reduzierung der Erscheinungsformen der Hautalterung bereitzustellen, die bislang marktübliche Produkte in der Leistung übertreffen. Es war eine weitere Aufgabe der vorliegenden Erfindung, eine neue, stabile und kosmetisch wirksame Hautpflegezubereitung zur Prophylaxe und Behandlung von Hautalterungserscheinungen, insbesondere Fältchen und Falten zu entwickeln. Insbesondere sollte durch die Hautpflegezubereitung die Hautfeuchtigkeit erhöht werden. Kosmetische oder dermatologische topische Zusammensetzungen, die eine verbesserte Behandlung von Fältchen, insbesondere von Fältchen, die auf die lichtinduzierte Hautalterung zurückzuführen sind, ermöglichen, werden in der Europäischen Patentanmeldung EP 1 634 576 A1 offenbart und enthalten in einem geeigneten kosmetischen oder dermatologischen Träger mindestens ein DNA-Reparaturenzym und mindestens ein Oligopeptid, das mindestens einen N-C₂-C₂₄-Acylrest aufweisen und/oder verestert sein kann. WO 2007/128723 offenbart die Verwendung von Benzylidenindanone als AhRezeptor Antagoniste zur Behandlung und Prophylaxe der Hautalterung. Es wurde nun festgestellt, dass die bekannten Zusammensetzungen, die mindestens ein DNA-Reparaturenzym enthalten, in ihren Eigenschaften verbessert werden können, wenn Ah-Rezeptor-Antagonisten in diese Mittel eingearbeitet wird. Die Abkürzung Ah-Rezeptor steht für "Aryl Hydrocarbon Receptor" bzw. Arylkohlenwasserstoff-Rezeptor. Dem Arylkohlenwasserstoff-Rezeptor (NCBI gene accession number BC0700800) kommt eine zentrale Rolle bei der Detoxifikation exogener Fremdstoffe zu. Der Ah-Rezeptor erfüllt eine Homöostasefunktion gegenüber lipidlöslichen Wirkstoffen, wie endogenen Stoffen wie Bilirubin oder Retinsäuren, Pflanzeninhaltsstoffen wie Polyphenolen und Flavonoiden, zahlreichen Arzneimitteln, aber auch carcinogenen polycyclischen aromatischen Kohlenwasserstoffen (PAK), wie Benzo[a]pyren. Diese lipidlöslichen Substanzen werden durch die Biotransformation in hydrophilere und damit ausscheidungsfähige Produkte umgewandelt. Die Aktivierung des Ah-Rezeptors kann aber nicht nur die Detoxifikation von Fremdstoffen bewirken, sondern auch deren toxische Wirkung auslösen, z. B. durch Bioaktivierung von Präcarcinogenen zu den eigentlichen elektrophilen Wirkformen. Als Folge der Aktivierung des Ah-Rezeptors können sich Tumore, Reizungen und Entzündungen der Haut, Allergien oder atopische Dermatitis ausbilden.

Verbindungen der nachstehenden Formel (I) stellen wirksame Antagonisten des Ah-Rezeptors dar.

Gegenstand der vorliegenden Erfindung ist in einer ersten Ausführungsform ein kosmetisches topisches Hautbehandlungsmittel, enthaltend in einem geeigneten Träger
a. mindestens eine Verbindung der Formel (I) 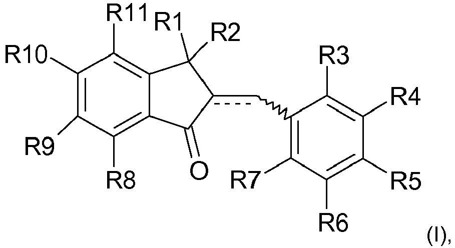 in der
   - die Reste R1 und R2 unabhängig voneinander für -H oder -CH₃ oder -CH₂CH₃ oder -CH(CH₃)₂ oder -(CH₂)ₙCH₃ mit n = 2 bis 11 stehen,
   - die Reste R3 bis R11 unabhängig voneinander für -H oder -CH₃ oder -CH₂CH₃ oder -CH(CH₃)₂ oder -(CH₂)ₖCH₃ mit k = 2 bis 11 oder -OH oder -OCH₃ oder -OCH₂CH₃ oder -OCH(CH₃)₂ oder -O(CH₂)ₘCH₃ mit m = 2 bis 11 stehen,
   - die gestrichelte Linie für eine Doppelbindung oder 2 Wasserstoffatome steht,
b. mindestens ein DNA-Reparaturenzym.

Erfindungsgemäß bevorzugt ist der Einsatz von Verbindungen der Formel (I), bei denen mindestens einer der Reste R1 oder R2 für eine Methylgruppe steht. Ganz besonders bevorzugte Hautbehandlungsmittel sind dadurch gekennzeichnet, dass sie mindestens eine Verbindung der Formel (I) enthalten, in der die Reste R1 und R2 für eine Methylgruppe stehen.

Die Reste R3, R6, R 8 und R11 der Formel (I) stehen vorzugsweise für -H, so dass in bevorzugten erfindungsgemäßen Mitteln für die darin enthaltenen Verbindungen der Formel (I) R1 = R2 = -CH₃ und gleichzeitig R3 = R6 = R8 = R11 = -H gilt.

Bevorzugte erfindungsgemäße Hautbehandlungsmittel sind dadurch gekennzeichnet, dass sie mindestens eine Verbindung der Formel (I) enthalten, in der die Reste R4, R5, R9 und R10 unabhängig voneinander ausgewählt sind aus -H oder -OCH₃, wobei Verbindngen bevorzugt sind, die insgesamt eine oder zwei -OCH₃ Gruppen im Molekül enthalten.

Der Rest R9 in der Formel (I) ist vorzugsweise -H, eine Methylgruppe oder eine Alkoxygruppe, vorzugsweise eine Methoxygruppe. Besonders bevorzugte erfindungsgemäße Hautbehandlungsmittel sind dadurch gekennzeichnet, dass sie mindestens eine Verbindung der Formel (I) enthalten, in der der Rest R9 eine Methylgruppe ist.

Der Rest R10 in der Formel (I) ist vorzugsweise -H, eine Methylgruppe oder eine Alkoxygruppe, vorzugsweise eine n-Butoxygruppe. Besonders bevorzugte erfindungsgemäße Hautbehandlungsmittel sind dadurch gekennzeichnet, dass sie mindestens eine Verbindung der Formel (I) enthalten, in der der Rest R10 eine -O(CH₂)₃CH₃ Gruppe ist.

Die gestrichelte Linie in Formel (I) steht vorzugsweise für eine Doppelbindung.

Im Rahmen der vorliegenden Erfindung haben sich einige Vertreter der Formel (I) als besonders geeignet erwiesen, da sie in Kobination mit der Komponente b) zu einer besonders hohen Leistungssteigerung führen. Erfindungsgemäß besonders bevorzugte Hautbehandlungsmittel, enthalten daher mindestens eine Verbindung der Formeln (Ia) bis (Ih):

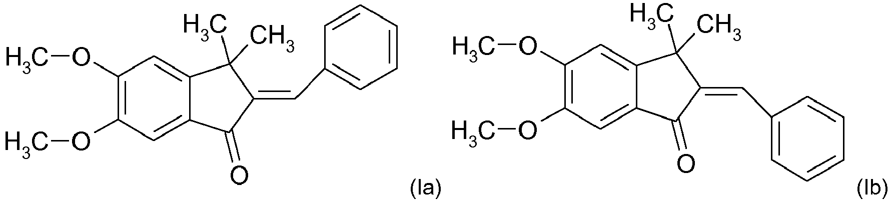

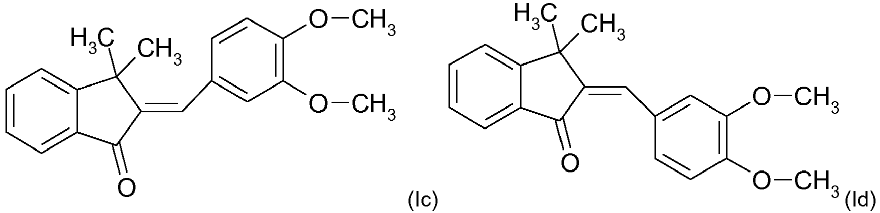

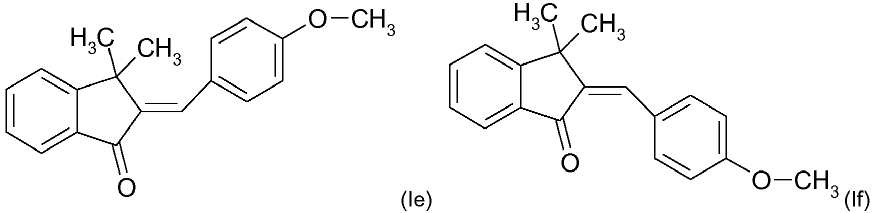

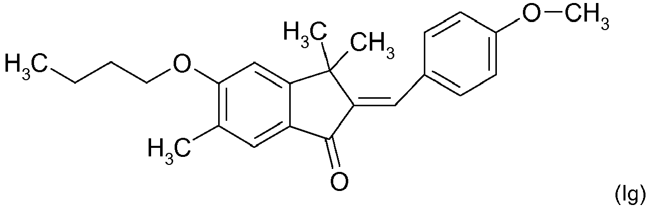

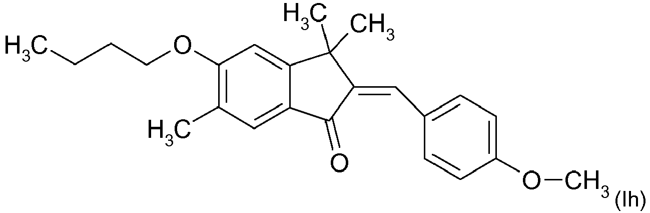

Unabhängig davon, ob die erfindungsgemäßen Mittel eine oder mehrere Verbindung(en) der Formel (I) enthalten und unabhängig von der speziellen Verbindung, werden die Gesamtmengen an Verbindungen der Formel (I) vorzugsweise innerhalb bestimmter Mengenbereiche gewählt. Hier sind erfindungsgemäße Hautbehandlungsmittel bevorzugt, die die Verbindung(en) der Formel (I) - bezogen auf das Gewicht des Hautbehandlungsmittels - in Mengen von 0,001 bis 10 Gew.-%, vorzugsweise von 0,005 bis 7,5 Gew.-%, weiter bevorzugt von 0,01 bis 5 Gew.-%, noch weiter bevorzugt von 0,025 bis 4 und insbesondere von 0,05 bis 3 Gew.-% enthalten. Ebenfalls bevorzugt sind erfindungsgemäße Hautbehandlungsmittel, die die Verbindung(en) der Formel (I) - bezogen auf das Gewicht des Hautbehandlungsmittels - in einer Gesamtmenge von 0,1 bis 1 Gew.-%, vorzugsweise von 0,15 bis 0,6 Gew.-%, weiter bevorzugt von 0,2 bis 0,5 Gew.-%, noch weiter bevorzugt von 0,25 bis 0,4 und insbesondere von 0,3 bis 0,35 Gew.-% enthalten.

Als zweiten wesentlichen Inhaltsstoff enthalten die erfindungsgemäßen Mittel mindestens ein DNA-Reparaturenzym. Als DNA-Reparaturenzyme haben sich im Rahmen der vorliegenden Erfindung insbesondere Photolyase, T4 Endonuclease V und 8-Oxoguanin-glycosylase bewährt. Bevorzugte erfindungsgemäße Hautbehandlungsmittel sind daher dadurch gekennzeichnet, dass sie als DNA-Reparatur-Enzym Photolyase enthalten. Ebenfalls bevorzugte erfindungsgemäße Hautbehandlungsmittel sind dadurch gekennzeichnet, dass sie als DNA-Reparatur-Enzym T4 Endonuclease V enthalten. Nicht zuletzt sind erfindungsgemäße Hautbehandlungsmittel bevorzugt, die als DNA-Reparatur-Enzym 8-Oxoguanin-glycosylase enthalten.
Ganz besonders bevorzugte erfindungsgemäße Zusammensetzungen sind dadurch gekennzeichnet, dass das DNA-Reparaturenzym ausgewählt ist aus Photolyase und T 4 Endonuclease V sowie Mischungen dieser Enzyme.
Die DNA-Reparaturenzyme können sowohl in Liposomen verkapselt als auch frei, das heißt unverkapselt, vorliegen. Die Liposomenverkapselung kann bevorzugt mit Phospholipiden, besonders bevorzugt mit Lecithin, erfolgen. Erfindungsgemäß besonders bevorzugt ist der Einsatz von mindestens einem liposomenverkapselten DNA-Reparaturenzym. Liposomenverkapselte Photolyase ist im Handel z. B. unter der Produktbezeichnung Photosomes™ (INCI-Bezeichnung: Aqua, Lecithin, Plankton Extract) liposomenverkapselte T4N5 z. B. unter der Bezeichnung Ultrasomes™ (INCI-Bezeichnung: Aqua, Lecithin, Micrococcus Lysate) von der Firma AGI Dermatics, USA, erhältlich. Da sich bei längerer Lagerung sowohl des Handelsproduktes als auch der erfindungsgemäßen Zusammensetzungen zwischen der verkapselten wässrigen, das Enzym enthaltenden Phase und der nicht-verkapselten, äußeren wässrigen Phase ein Gleichgewicht einstellt, liegt ein Teil der DNA-Reparaturenzyme unverkapselt vor.

In den erfindungsgemäßen Zusammensetzungen sind die Handelsprodukte Photosomes™ oder Ultrasomes™ bevorzugt in Mengen von 0,1 - 10 Gew.-%, besonders bevorzugt 0,5 - 5,0 Gew.-% und außerordentlich bevorzugt 1,0 - 4,0 Gew.-%, bezogen auf das gesamte Mittel, enthalten.

Bevorzugte erfindungsgemäße Zusammensetzungen sind dadurch gekennzeichnet, dass mindestens ein DNA-Reparaturenzym in Gesamtmengen von 0,00001 - 1 Gew.-%, bevorzugt 0,0001 - 0,1 Gew.-%, besonders bevorzugt 0,001 - 0,01 - 0,05 Gew.-% und außerordentlich bevorzugt 0,002 - 0,005 Gew.-%, enthalten ist.

Ganz besonders bevorzugte erfindungsgemäße Hautbehandlungsmittel sind dadurch gekennzeichnet, dass sie das/die DNA-Reparaturenzym(e) - bezogen auf das Gewicht des Hautbehandlungsmittels - in Mengen von 0,00001 - 1 Gew.-%, bevorzugt 0,0001 - 0,1 Gew.-%, besonders bevorzugt 0,001 -0,05 Gew.-% und außerordentlich bevorzugt 0,002 - 0,005 Gew.-%, enthalten. Die erfindungsgemäßen Zusammensetzungen können darüber hinaus Antioxidationsmittel enthalten, was erfindungsgemäß besonders bevorzugt ist. Im Rahmen der vorliegenden Erfindung haben sich einige Antioxidationsmittel als besonders leistungsstark in Kombination mit den AhRezeptor-Antagonisten erwiesen, so dass deren Einsatz bevorzugt ist. Bevorzugte erfindungsgemäße Hautbehandlungsmittel enthalten zusätzlich mindestens ein Antioxidationsmittel aus der Gruppe
- der 6,7-disubstituierten 2,2-Dialkylchromanen oder -chromenen der allgemeinen Formeln (II) oder (III), 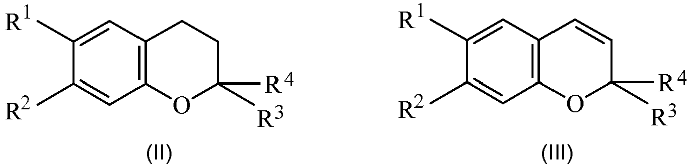 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen, insbesondere Lipochroman-6 und/oder
- der Ubichinone, insbesondere Coenzym Q10, und/oder
- der Quercetine und/oder
- Ellagsäure und/oder Ellagate und/oder
- der Hydroxystilben-Oligomeren, deren Alkylethern und/oder deren Estern und/oder
- der Xanthophylle, insbesondere Astaxanthin, und/oder
- der Superoxiddismutasen.

Diese Stoffe werden nachstehend beschrieben.

Als erstes besonders geeignetes Antioxidans können die erfindungsgemäßen Mittel 6,7-disubstituierte 2,2-Dialkylchromane oder -chromene der allgemeinen Formeln (II) oder (III), 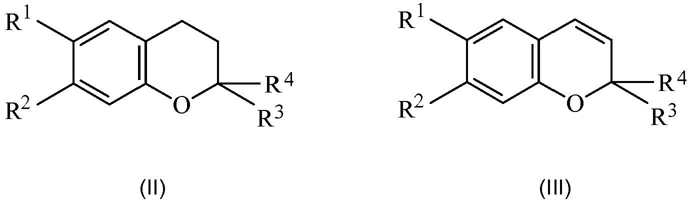 enthalten, wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen. Die Substituenten R¹ und R² sind unabhängig voneinander ausgewählt aus einer OH-Gruppe, einer Methoxy-Gruppe und einer CF₃CH₂O-Gruppe. In einer bevorzugten Ausführungsform sind R¹ und R² unabhängig voneinander ausgewählt aus einer OH-Gruppe und einer Methoxy-Gruppe. In einer besonders bevorzugten Ausführungsform stellen R¹ eine OH-Gruppe und R² eine Methoxy-Gruppe dar.

Die Substituenten R³ und R⁴ stellen unabhängig voneinander eine C₁-C₄-Alkylgruppe dar. Unter C₁-C₄-Alkylgruppe ist dabei erfindungsgemäß eine Methyl-, Ethyl-, n-Propyl-, Isopropyl-, n-Butyl-, Isobutyl- beziehungsweise 2-Methylpropyl-, sec-Butyl- beziehungsweise 1-Methylpropyl- oder eine tert.-Butyl-Gruppe zu verstehen. In einer bevorzugten Ausführungsform sind R³ und R⁴ unabhängig voneinander ausgewählt aus einer Methyl-, Ethyl-, n-Propyl-, Isopropyl- und einer n-Butyl-Gruppe. Besonders bevorzugt stellen R³ und R⁴ unabhängig voneinander eine Methyl- oder Ethylgruppe dar. Außerordentlich bevorzugt ist, dass R³ und R⁴ identisch sind.

Erfindungsgemäß bevorzugt verwendet werden die 6,7-disubstituierten 2,2-Dialkylchromane. Ein erfindungsgemäß außerordentlich bevorzugt verwendeter Wirkstoff ist 2,2-Dimethyl-6-hydroxy-7-methoxy-chroman mit der systematischen Bezeichnung 3,4-Dihydro-7-methoxy-2,2-dimethyl-2H-1-benzopyran-6-ol und der INCI-Bezeichnung Dimethylmethoxy Chromanol. Die Substanz ist unter dem Handelsnamen Lipochroman-6 von der Firma Lipotec S.A. erhältlich.

Die 6,7-disubstituierten 2,2-Dialkylchromane oder -chromene der allgemeinen Formeln (II oder (II) werden vorzugsweise in Mengen von 0,001 - 5 Gew.-%, bevorzugt 0,005 - 2 Gew.-% und besonders bevorzugt 0,01 - 0,1 Gew.-%, jeweils bezogen auf die gesamte Zusammensetzung, eingesetzt. Zusammenfassend sind erfindungsgemäße Hautbehandlungsmittel bevorzugt, die - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% 6,7-disubstituierte 2,2-Dialkylchromane oder -chromene der allgemeinen Formeln (II) oder (III) wie vorstehend definiert, enthalten, wobei bevorzugte Mittel - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% Lipochroman-6 enthalten. Weitere erfindungsgemäß bevorzugte Zusammensetzungen enthalten - bezogen auf ihr Gewicht - 3,4-Dihydro-7-methoxy-2,2-dimethyl-2H-1-benzopyran-6-ol in einer Gesamtmenge von 0,001 - 1 Gew.-%, bevorzugt 0,005 - 0,5 Gew.-%, besonders bevorzugt 0,01 - 0,2 Gew.-%, außerordentlich bevorzugt 0,05 - 0,1 Gew.-%.

Als ein weiteres besonders geeignetes Antioxidans können die erfindungsgemäßen Mittel bestimmte Chinone enthalten. Als weiteren Bestandteil können die erfindungsgemäßen Mittel daher 0,0001 bis 5 Gew.-% mindestens eines Biochinons der Formel (Ubi) 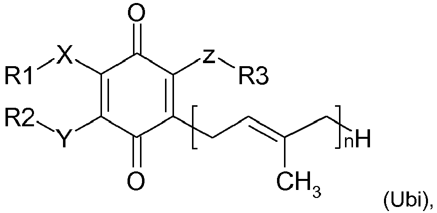 enthalten, wobei gilt:
- X, Y, Z: stehen unabhängig voneinander für -O- oder -NH- oder NR⁴- oder eine chemische Bindung,
- R¹, R², R³: stehen unabhängig voneinander für ein Wasserstoffatom oder eine gegebenenfalls substituierte Arylgruppe oder eine gegebenenfalls substituierte (C₁-C₆)-Alkyl-gruppe oder eine Hydroxyalkylgruppe oder eine Polyhydroxyalkylgruppe oder eine gegebenenfalls substituierte (C₁-C₆)-Alkylengruppe, oder einen (C₁-C₆)-Acylrest, wobei bevorzugte Reste unabhängig voneinander ausgewählt sind aus -H, -CH₃, -CH₂CH₃, -(CH₂)₂CH₂, -CH(CH₃)₂, -(CH₂)₃CH₃, -CH(CH₃)CH₂CH₃, -CH₂CH(CH₃)₂, -C(CH₃)₃
- R⁴: steht für -CH₃, -CH₂CH₃, -(CH₂)₂CH₂, -CH(CH₃)₂, -(CH₂)₃CH₃, -CH(CH₃)CH₂CH₃, -CH₂CH(CH₃)₂, -C(CH₃)₃
- n: steht für Werte von 1 bis 20, vorzugsweise von 2 bis 15 und insbesondere für 5, 6, 7,8,9, 10.

Erfindungsgemäß bevorzugte Verbindungen der Formel (Ubi), sind beispielsweise 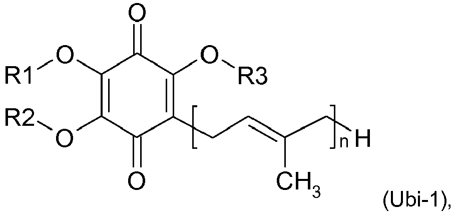 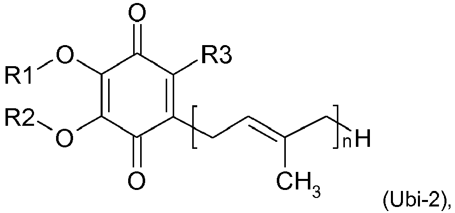 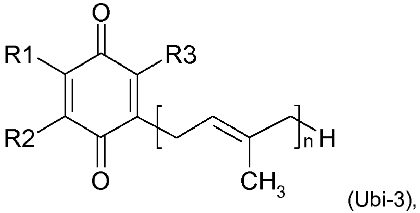 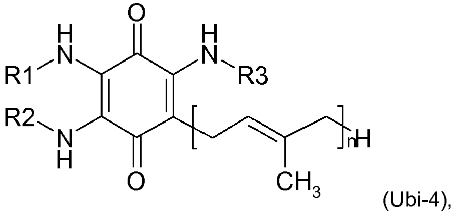 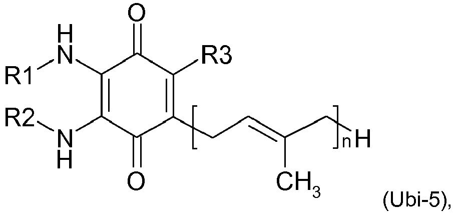 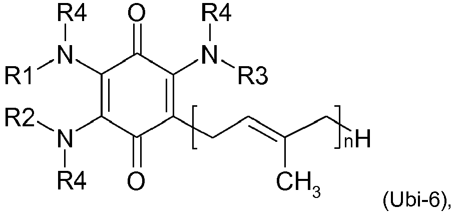 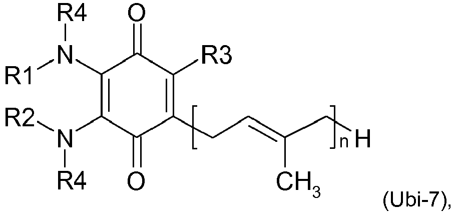 wobei gilt:
- R¹, R², R³: stehen jeweils unabhängig voneinander für -H, -CH₃, -CH₂CH₃, -(CH₂)₂CH₂, -CH(CH₃)₂, -(CH₂)₃CH₃, -CH(CH₃)CH₂CH₃, -CH₂CH(CH₃)₂, -C(CH₃)₃
- R⁴: steht für -CH₃, oder -CH₂CH₃, oder -(CH₂)₂CH₂, oder -CH(CH₃)₂
- n: steht für Werte von 1 bis 20, vorzugsweise von 2 bis 15 und insbesondere für 5, 6, 7, 8, 9, 10.

Besonders bevorzugte erfindungsgemäße Hautbehandlungsmittel sind dadurch gekennzeichnet, dass sie- bezogen auf ihr Gewicht - 0,0001 bis 1 Gew.-%, bevorzugt 0,001 bis 0,5 Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei bevorzugte Mittel ein Ubichinon der Formel (Ubi) enthalten 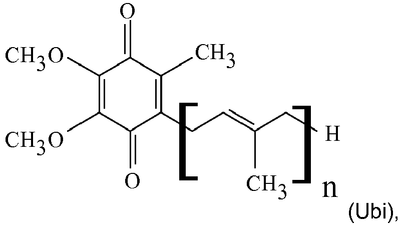 in der n für die Werte = 6, 7, 8, 9 oder 10, besonders bevorzugt für 10 (Coenzym Q10) steht.

Alternativ zu den besonders bevorzugten Ubichinonen oder zusätzlich zu ihnen können die erfindungsgemäßen Mittel auch Plastochinone enthalten. Hier sind bevorzugte erfindungsgemäße Mittel dadurch gekennzeichnet, dass sie 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% mindestens eines Plastochinons der Formel (Ubi-Ib) enthalten 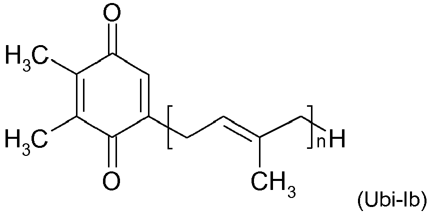 in der n für Werte von 1 bis 20, vorzugsweise von 2 bis 15 und insbesondere für 5, 6, 7, 8, 9, 10 steht, wobei besonders bevorzugt Mittel Plastochinon PQ-9 der Formel 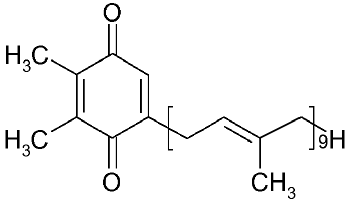 enthalten.

Als ein weiteres besonders geeignetes Antioxidans können die erfindungsgemäßen Mittel mindestens einen Stoff aus der Gruppe der Quercetine enthalten. Bevorzugte Quercetine sind vorzugsweise ausgewählt aus der Gruppe, umfassend 3,3',4',5,7-Pentahydroxyflavanone, 3,3',4',5,7-Pentahydroxyflavon 3-β-glucoside (Isoquercitrin), Quercetin-7-methylether, Quercetin-3',7-dimethylether, Quercetin-3-*O*-β-D-glucofuranoside, Quercetin-7-*O*-glucoside, Quercetin-4'-*O-*glucoside, Quercetin-3-*O*-galactoside, Quercetin-3-D-xyloside, Quercetin-3-rhamnoside (Quercitrine), Quercetin-3-rutinoside, Dihydroquercetine und Mischungen daraus.

Vorzugsweise werden die Quercetinderivate als Extrakte gewonnen. Beispielsweise kann Quercetin-Glycoside aus Rinden und Schale vieler Früchte und Gemüsearten gewonnen werden. Quercetin-7-methylether, Quercetin-3',7-dimethylether kommen beispielsweise in Rosskastanien, Zwiebel, Kamille Stiefmütterchen, Eichen und Rosen vor. Daher ist der Einsatz von Quercetinen als Quercetin-haltige Extrakte ebenfalls geeignet, bevorzugt dabei werden Dihydroquercetinhaltige Extrakte eingesetzt.

Die Extrakte von Quercetinen können mit Wasser, sowie polaren oder unpolaren organischen Lösungsmitteln sowie Mischungen davon in dem Fachmann bekannter Weise hergestellt werden. Extrakte, die durch Extraktion mit Ethanol oder Wasser/Ethanol-Mischungen, erhalten werden können, sowie Presssaft, sind bevorzugt.

Es können sowohl die Extrakte im ursprünglichen Extraktionsmittel als auch Extrakte/Presssaft in Wasser oder anderen organischen Lösungsmitteln und/oder deren Gemisch, insbesondere Ethanol sowie Ethanol/Wasser-Mischungen eingesetzt werden. Bevorzugt wird extrahiertes oder gepresstes Material als Feststoff eingesetzt, dem das Lösungsmittel (insbesondere möglichst schonend) entzogen wurde. Es können aber auch solche Extrakte/Presssäfte eingesetzt werden, denen das Lösungsmittel zum Teil entzogen wurde, so dass ein verdickter Extrakt/Presssaft eingesetzt wird.

Vorzugsweise wird ein Extrakt der Firma Bioflavon Ltd. namens Flavocon eingesetzt. Dieser Extrakt der Sibirischen Lärche enthält ca. 98 % Flavonoide, wobei davon ca. 90 % aus Dihydroquercetin bestehen.

Zusammenfassend sind erfindungsgemäße Hautbehandlungsmittel bevorzugt, die - bezogen auf ihr Gewicht - 0,00001 bis 5 Gew.-%, vorzugsweise 0,0001 bis 2,5 Gew.-%, besonders bevorzugt 0,0001 bis 1 Gew.-% und insbesondere 0,00025 bis 0,5 Gew.-% Quercetine oder Quercetin-haltige Extrakte enthalten.

In bevorzugten erfindungsgemäßen Hautbehandlungsmittel sind die Quercetine ausgewählt aus der Gruppe, umfassend 3,3',4',5,7-Pentahydroxyflavanone, 3,3',4',5,7-Pentahydroxyflavon 3-β-glucoside (Isoquercitrin), Quercetin-7-methylether, Quercetin-3',7-dimethylether, Quercetin-3-*O-*β-D-glucofuranoside, Quercetin-7-*O*-glucoside, Quercetin-4'-*O*-glucoside, Quercetin-3-*O*-galactoside, Quercetin-3-D-xyloside, Quercetin-3-rhamnoside (Quercitrine), Quercetin-3-rutinoside, Dihydroquercetine und Mischungen daraus.

Als ein weiteres besonders geeignetes Antioxidans können die erfindungsgemäßen Mittel mindestens einen Stoff aus der Gruppe der Ellagsäure und/oder Ellagate enthalten. Ellagsäure (2,3,7,8-Tetrahydroxy[1]benzopyrano-[5,4,3-*cde*][1]benzopyran-5,10-dion 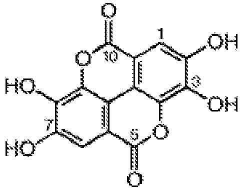 ist frei und in Form von Glycosiden und/oder Methylethern im Pflanzenreich weit verbreitet, besonders in Galläpfeln, Blattgallen, auch in den Blättern von *Eucalyptus-Arten,* in Eichen und Euphorbien.

Bevorzugte erfindungsgemäße Hautbehandlungsmittel sind dadurch gekennzeichnet, dass sie - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% Ellagsäure (2,3,7,8-Tetrahydroxy[1]benzopyrano-[5,4,3-*cde*][1]benzopyran-5,10-dion) und/oder Ellagate enthalten.

Als ein weiteres besonders geeignetes Antioxidans können die erfindungsgemäßen Mittel mindestens einen Stoff aus der Gruppe der Hydroxystilben-Oligomeren, deren Alkylethern und/oder deren Estern enthalten.

Erfindungsgemäß besonders bevorzugt wird die mindestens eine Verbindung, ausgewählt aus den Hydroxystilben-Oligomeren, deren Alkylethern und/oder deren Estern, in einer Gesamtmenge von 0,00001 - 10 Gew.-%, bevorzugt 0,0001 - 1 - 5 Gew.-%, besonders bevorzugt 0,001 - 0,5-4 Gew.-%, außerordentlich bevorzugt 0,005 - 0,1 - 1 Gew.-%, jeweils bezogen auf das Gesamtgewicht der erfindungsgemäß verwendeten Zusammensetzung, verwendet.

Bei Hydroxystilbenen handelt es sich um eine umfangreiche Gruppe von Naturstoffen, meist Phytoalexine; viele davon werden aus Koniferen gewonnen und liegen häufig als Glycoside und/oder Methylether vor. Sie können aber auch synthetisch hergestellt werden. Auch mit Säuren, beispielsweise mit Phosphorsäure, veresterte Hydroxystilbene sind für die erfindungsgemäßen Verwendungen geeignet. Die meisten Hydroxystilbene liegen in der (E)-Form vor, einige kommen aber auch in der (Z)-Form vor. Zu den wichtigsten Vertretern zählt Pinosylvin (3,5-Stilbendiol). In der (E)-Form bildet Pinosylvin Nadeln, die in Wasser unlöslich, in Aceton löslich sind. (E)- u. (Z)-Isomere kommen neben den entsprechenden Mono- und Dimethylethern in Pinien, Kiefern und anderen Nadelhölzern vor. Pinosylvin besitzt fungizide und antibakterielle Eigenschaften. Pinosylvin-monomethylether wirkt als Fraßhemmstoff. Resveratrol (3,4',5-Stilbentriol, 3,4',5-Trihydroxystilben, Struktur siehe allgemeine Formel RESV-I, mit R¹ = OH und R² = H) ist ein Phytoalexin aus den Wurzeln von Veratrum grandiflorum und der Rinde von Pinus sibirica und kommt unter anderem in Eucalyptus-, Polygonum- u. Nothofagus-Arten vor, insbesondere in dem Rhizom von Polygonum cuspidatum. Piceatannol (3,3',4,5'-Stilbentetrol, 3,3',4,5'-Tetrahydroxystilben, Astringenin) bildet in der (E)-Form Nadeln, wurde aus Fichten isoliert und ist ebenfalls fungitoxisch, hemmt das Pflanzenwachstum und wirkt ichthyotoxisch.

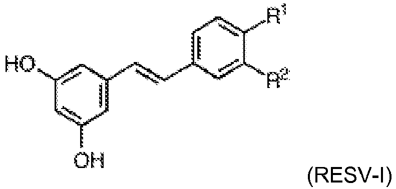

Über den Angriff einer Hydroxylgruppe an die ethylenische Doppelbindung eines weiteren Hydroxystilben-Moleküls unter Ausbildung eines Dihydrofuranrings können Hydroxystilbene oligomerisieren. Bekannte Beispiele für die Oligomere von Hydroxystilbenen sind die Dimere des Resveratrols, insbesondere des (E)-Resveratrols, sind die sogenannten Viniferine, insbesondere das trans-epsilon-Viniferin (siehe Formel RESV-II).

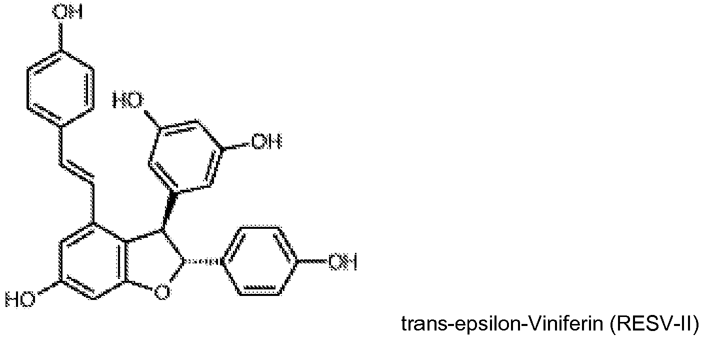

Bekannte Trimere und Tetramere des Resveratrols sind Vitisin E (siehe Formel RESV-III) und Vitisin D (siehe Formel RESV-IV).

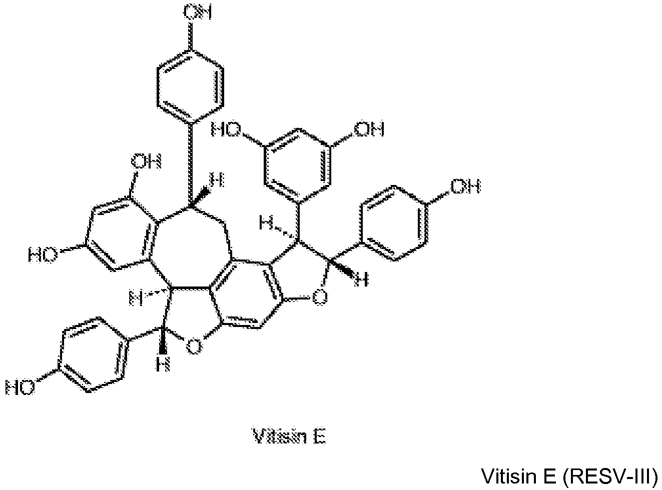

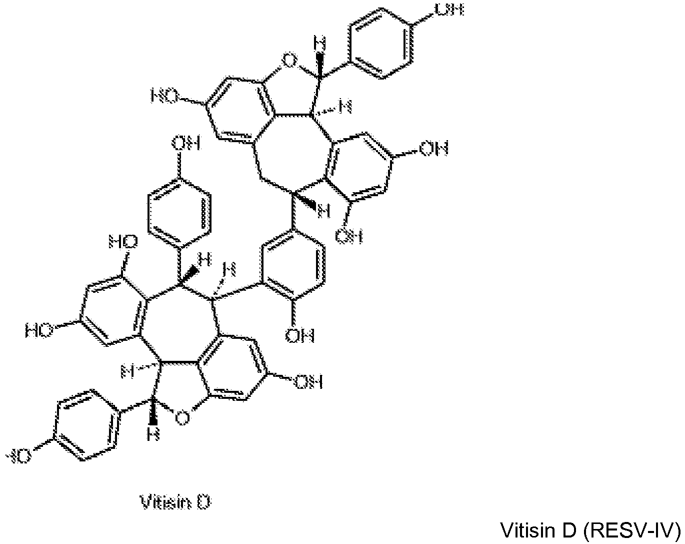

Die Summenformel von trans-epsilon-Viniferin lautet C₂₈H₂₂O₆ (CAS: 62218-08-0). Man findet diese Substanz in freier Form oder als Glycosid in variablen Mengen in diversen Pflanzenarten, darunter Vitaceae, Umbelliferae, Myrtaceae, Dipterocarpaceae, Cyperaceae, Gnetaceae, Leguminosen, Gramineae, Sericeae, Haemodoraceae, Musaceae, Polygonaceae, Pinaceae, Cupressaceae, Cesalpiniaceae, Poaceae, et Solanaceae, insbesondere in Balanocarpus zeylanicus, Caragana chamlagu, Caragana sinica, Carex fedia, Carex humilis, Carex kobomugi, Carex pendula, Carex pumila Thunb, Cyphostemma crotalarioides, Gnetum hainanense, Gnetum ula, Gnetum venosum, Hopea parviflora, Iris clarkei, Neobalanocarpus heimii, Paeoni lactiflora, Parthenocissus tricuspidata, Polygonum cuspidatum, Polygonum multiflorum, Shorea disticha, Shorea hemsleyana, Sophora davidii, Sophora leachiana, Sophora nuttaliana, Vatica rassak, Vatica affinis, Vitis amurensis, Vitis betulifolia, Vitis flexuosa, Vitis heyneana, Vitis quinquangularis, Vitis coignetiae, Vitis vinifera. Im Stand der Technik bekannt ist die hormonähnliche Wirkung des trans-epsilon-Viniferins, die wiederum der Wirkung der Retinoide ähnelt.

Besonders bevorzugt werden die Hydroxystilben-Oligomere aus den Vitaceae extrahiert, insbesondere aus den Stielen von Weintrauben, da diese mengenmäßig gut verfügbar sind. Als Extraktionsmittel eignen sich sowohl Wasser als auch organische Lösemittel. Bevorzugte organische Lösemittel sind Ethylacetat und/oder Diethylether. Störende Lipide, die ebenfalls mit extrahiert werden, können beispielsweise mittels Petrolether, Hexan oder Chloroform entfernt werden. Bevorzugte Extraktionsverfahren erfolgen mit Hilfe organischer Lösemittel und Mikrowellen- oder Ultraschall-Behandlung, oder mittels Maceration-Lixiviation oder mittels Extraktion durch superkritische Fluide. Die so angereicherten Extrakte werden vorteilhafterweise filtriert, gewaschen und besonders bevorzugt gefriergetrocknet.

Unter den Oligomeren von Hydroxystilbenen werden erfindungsgemäß Dimere, Trimere, Tetramere, Pentamere und Hexamere von Hydroxystilbenen verstanden. Besonders bevorzugt sind die Dimere, Trimere und Tetramere, außerordentlich bevorzugt sind die Dimere von Hydroxystilbenen. Die Oligomerisierung erfolgt erfindungsgemäß besonders bevorzugt durch den Angriff einer Hydroxylgruppe an die ethylenische Doppelbindung eines weiteren Hydroxystilben-Moleküls unter Ausbildung eines Dihydrofuranrings, wie es oben exemplarisch am Beispiel von (E)-Resveratrol und den daraus gebildeten Oligomeren trans-epsilon-Viniferin, Vitisin E und Vitisin D beschrieben ist. Prinzipiell sind aber auch andere Oligomerisierungsreaktionen möglich und können bevorzugt sein, beispielsweise die Kondensation von zwei und mehr Hydroxystilben-Molekülen über die Hydroxylgruppen unter Bildung von Etherbindungen. Da die durch Kondensation gebildeten Oligomere sterisch weniger stark gehindert sind als die Oligomere, die an der ethylenischen Stilben-Doppelbindung entstehen, sind hierbei neben den Dimeren, Trimeren, Tetrameren, Pentameren und Hexameren auch höhere Oligomere, beispielsweise Heptamere und Octamere, möglich. Die Hydroxystilben-Oligomere können aus gleichen Monomeren (= Homooligomerisierung) oder aus verschiedenen Monomeren (= Heterooligomerisierung) gebildet sein. Erfindungsgemäß bevorzugt sind die Homooligomere, bezogen auf die nicht-derivatisierten, das heißt beispielsweise, nicht veretherten oder nicht-veresterten, Hydroxystilben-Monomere. Die Derivatisierung der Hydroxystilbene, beispielsweise die Veretherung oder Veresterung der OH-Gruppen, kann vor oder nach der Oligomerisierung erfolgen. Die Derivatisierung nach der Oligomerisierung kann zu einem unsymmetrischen Substitutionsmuster führen.

Erfindungsgemäß besonders bevorzugt verwendete Hydroxystilben-Oligomere sind aus Hydroxystilben-Monomeren der allgemeinen Formel (RESV-I a) und (RESV-I b) gebildet, 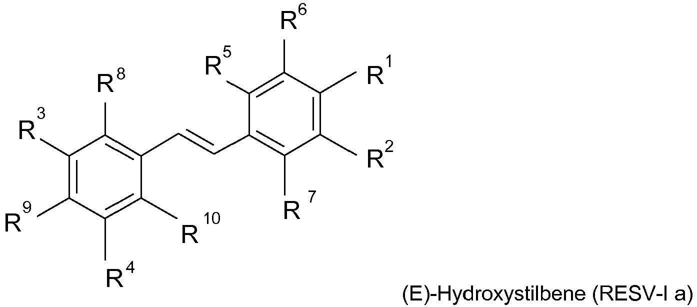 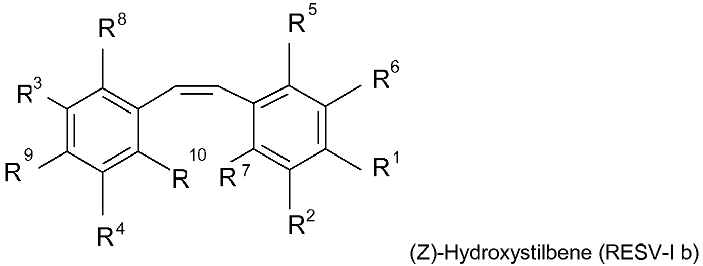 wobei die Reste R¹ bis R¹⁰ unabhängig voneinander ein Wasserstoffatom, eine OH-Gruppe, eine Alkylgruppe, die bevorzugt eine C₁-C₄-Alkylgruppe darstellt, eine Alkoxygruppe, die bevorzugt einen C₁-C₄-Alkylrest trägt, ein Glycosid-Rest, eine ganz oder teilweise neutralisierte Phosphatgruppe -OPO₃, eine ganz oder teilweise neutralisierte Sulfatgruppe -OSO₃ oder eine Estergruppe -OCOR' mit R'COOH = organische Säure sein können, wobei mindestens einer der Reste R¹ bis R¹⁰ ausgewählt ist aus einer OH-Gruppe, einer Alkoxygruppe, die bevorzugt einen C₁-C₄-Alkylrest trägt, einem Glycosid-Rest, einer ganz oder teilweise neutralisierten Phosphatgruppe -OPO₃, einer ganz oder teilweise neutralisierten Sulfatgruppe -OSO₃ und einer Estergruppe -OCOR' mit R'COOH = organische Säure.

Die Alkylgruppe in den Formeln RESV-I a und RESV-I b ist erfindungsgemäß bevorzugt ausgewählt aus einer Methyl-, Ethyl-, n-Propyl-, i-Propyl-, n-Butyl-, sec-Butyl-, tert.-Butyl-Gruppe und 2-Ethylhexyl-Gruppe, besonders bevorzugt ausgewählt aus einer Methyl-Gruppe.

Die Alkoxygruppe in den Formeln RESV-I a und RESV-I b ist erfindungsgemäß bevorzugt ausgewählt aus einer Methoxy-, Ethoxy-, n-Propoxy-, i-Propoxy-, n-Butoxy-, sec-Butoxy-, tert.-ButoxyGruppe und 2-Ethylhexoxy-Gruppe, besonders bevorzugt ausgewählt aus einer Methoxy-Gruppe. Der Glycosid-Rest in den Formeln RESV-I a und RESV-I b ist erfindungsgemäß bevorzugt ausgewählt aus einem Rest, abstammend von D-Glucose, Fructose, D-Galactose, L-Arabinose, Ribose, D-Xylose, Lyxose, Allose, Altrose, Mannose, Gulose, Idose, Talose, Fucose, und Sucrose, wobei D-Glucose, D-Apiose, L-Rhamnose, L-Rhamnoglycosiden, Rutinose (6-O-alpha-L-Rhamnopyranosyl-D-glucose), D-Glucuronsäure, D-Galacturonsäure und Neohesperidose besonders bevorzugt sind.

Die organische Säure R'COOH, die zur Veresterung der in den Formeln RESV-I a und RESV-I b dargestellten Verbindungen verwendet wird, ist erfindungsgemäß bevorzugt ausgewählt aus den linearen und verzweigten, gesättigten, einfach ungesättigten und mehrfach ungesättigten, ggf. aromatischen C₁-C₃₀-Monocarbonsäuren, insbesondere Ameisensäure, Essigsäure, Propansäure, Butansäure, Pentansäure, Valeriansäure, Isovaleriansäure, Hexansäure, Sorbinsäure ((*E*,*E*)-2,4-Hexadiensäure), 2-Ethylhexansäure, Heptansäure, Benzoesäure, Octansäure, Nonansäure, Decansäure, Undecansäure, Laurinsäure, Tridecansäure, Tetradecansäure, Myristinsäure, Palmitinsäure, Palmitoleinsäure, Stearinsäure, Ölsäure, Elaidinsäure, Petroselinsäure ((Z)-6-Octadecensäure), Linolsäure, Linolensäure, Arachinsäure, Arachidonsäure, (all-Z)-5,8,11,14,17-Eicosapentaensäure, Behensäure, Erucasäure, (all-Z)-4,7,10,13,16,19-Docosahexaensäure, weiterhin bevorzugt ausgewählt aus den linearen und verzweigten, gesättigten, einfach ungesättigten und mehrfach ungesättigten, ggf. aromatischen C₂-C₂₀-Hydroxymono-, -di- und tricarbonsäuren, insbesondere Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Ricinolsäure, Mandelsäure (Hydroxyphenylessigsäure), 4-Hydroxymandelsäure, Äpfelsäure (Hydroxybernsteinsäure), Erythrarsäure, Threarsäure, Glucarsäure, Galactarsäure, Mannarsäure, Gularsäure, 2-Hydroxy-2-methylbernsteinsäure, Gluconsäure, Glucuronsäure, Galacturonsäure und Salicylsäure, wobei Hexansäure, Sorbinsäure ((*E*,*E*)-2,4-Hexadiensäure), 2-Ethylhexansäure, Octansäure, Decansäure, Laurinsäure, Myristinsäure, Palmitinsäure, Stearinsäure, Glycolsäure und Milchsäure besonders bevorzugt sind.

Erfindungsgemäß besonders bevorzugt sind die Oligomere der (E)-Hydroxystilbene, insbesondere die Dimere der (E)-Hydroxystilbene. Erfindungsgemäß besonders bevorzugt verwendete Hydroxystilben-Oligomere sind aus folgenden Hydroxystilben-Monomeren gebildet:
4'-Hydroxystilben (R¹ = OH),
2',4'-Dihydroxystilben (R¹ = R⁵ = OH),
3',4'-Dihydroxystilben (R² = R⁵ = OH),
4,4'-Dihydroxystilben (R¹ = R⁹ = OH),
3,5- Dihydroxystilben (Pinosylvin) (R³ = R⁴ = OH),
3-Hydroxy-5-methoxystilben (Pinosylvinmonomethylether, (R³ = OH, R⁴ = -OCH₃)
2',4',4-Trihydroxystilben (R¹ = R⁵ = R⁹ = OH),
3',4',4-Trihydroxystilben (R² = R⁵ = R⁹ = OH),
2,4,4'-Trihydroxystilben (R¹ = R⁸ = R⁹ = OH),
3,4,4'-Trihydroxystilben (R¹ = R³ = R⁹ = OH),
3,4',5-Trihydroxystilben (Resveratrol) (R¹ = R³ = R⁴ = OH),
2',3,4-Trihydroxystilben (R¹ = R⁵ = R⁶ = OH),
2,3',4-Trihydroxystilben (R⁵ = R⁶ = R⁸ = OH),
2',2,4'-Trihydroxystilben (R¹ = R⁵ = R⁸ = OH),
2,4,4',5-Tetrahydroxystilben (R¹ = R⁴ = R⁸ = R⁹ = OH),
2',3,4',5-Tetrahydroxystilben (R¹ = R³ = R⁴ = R⁵ = OH),
2,2',4,4'-Tetrahydroxystilben (R¹ = R⁵ = R⁸ = R⁹ = OH),
3,3',4',5-Tetrahydroxystilben (Piceatannol) (R¹ = R³ = R⁴ = R⁶ = OH),
2,3',4,4'-Tetrahydroxystilben (R¹ = R⁶ = R⁸ = R⁹ = OH),
3,3',4,4'-Tetrahydroxystilben (R¹ = R³ = R⁶ = R⁹ = OH),
3,3',4',5,5'-Pentahydroxystilben (R¹ = R² = R³ = R⁴ = R⁶ = OH),
2,2',4,4',6-Pentahydroxystilben (R¹ = R⁵ = R⁸ = R⁹ = R¹⁰ = OH),
2,3',4,4',6-Pentahydroxystilben (R¹ = R⁶ = R⁸ = R⁹ = R¹⁰ = OH),
2,2',4,4',6,6'-Hexahydroxystilben (R¹ = R⁵ = R⁷ = R⁸ = R⁹ = R¹⁰ = OH),
wobei für die nicht genannten R^{x} = H gilt, das heißt, alle übrigen Substituenten R stellen ein Wasserstoffatom dar, und wobei alle Verbindungen sowohl in der (E)-Form als auch in der (Z)-Form vorliegen, wobei die Verbindungen, die in der (E)-Form vorliegen, besonders bevorzugt sind.

Erfindungsgemäß besonders bevorzugt verwendete Hydroxystilben-Oligomere sind ausgewählt aus trans-epsilon-Viniferin, Vitisin E und Vitisin D sowie Mischungen hiervon, weiterhin ausgewählt aus Mono-, Di-, Tri-, Tetra- und Pentamethoxy-trans-epsilon-Viniferin, trans-epsilon-Viniferin-Mono-, Di-, Tri-, Tetra- und Pentaalkanoaten, insbesondere trans-epsilon-Viniferin-Mono-, Di-, Tri-, Tetra- und Pentapalmitat, Mono-, Di-, Tri-, Tetra-, Penta-, Hexa- und Heptamethoxy-Vitisin E, Vitisin E-Mono-, Di-, Tri-, Tetra-, Penta-, Hexa- und Heptaalkanoaten, insbesondere Vitisin E-Mono-, Di-, Tri-, Tetra-, Penta-, Hexa- und Heptapalmitat, Mono-, Di-, Tri-, Tetra-, Penta-, Hexa-, Hepta-, Octa-, Nona- und Decamethoxy-Vitisin D, Vitisin D-Mono-, Di-, Tri-, Tetra-, Penta-, Hexa-, Hepta-, Octa-, Nona- und Decaalkanoaten, insbesondere Vitisin D-Mono-, Di-, Tri-, Tetra-, Penta-, Hexa-, Hepta-, Octa-, Nona- und Decapalmitat, sowie Mischungen hiervon. Erfindungsgemäß bevorzugte Hautbehandlungsmittel enthalten - bezogen auf ihr Gewicht - 0,01 bis 10 Gew.-%, vorzugsweise 0,05 bis 7,5 Gew.-%, weiter bevorzugt 0,075 bis 5 Gew.-% und insbesondere 0,1 bis 4 Gew.-% Weinrebenextrakt(e).

Als ein weiteres besonders geeignetes Antioxidans können die erfindungsgemäßen Mittel mindestens einen Stoff aus der Gruppe der Xanthophylle, insbesondere Astaxanthin, enthalten.

Xanthophylle ist der Gruppenname für Hydroxy-, Epoxy- und Oxo-Derivate von Carotinoiden (= Tetraterpenen mit einem C₄₀-Grundgerüst). Beispiele für erfindungsgemäß geeignete Verbindungen sind z. B. Canthaxanthin, Citranaxanthin, Flavoxanthin, Rhodoxanthin, Rubixanthin, Zeaxanthin, Lutein, Cryptoxanthin, Violaxanthin und insbesondere Astaxanthin. Die natürlich vorkommenden Xanthophylle sind meist *all-trans*-Verbindungen.

Erfindungsgemäß bevorzugte Hautbehandlungsmittel enthalten - bezogen auf ihr Gewicht - 0,001 bis 10 Gew.-%, vorzugsweise 0,005 bis 7,5 Gew.-%, weiter bevorzugt 0,01 bis 5 Gew.-% und insbesondere 0,02 bis 1 Gew.-%, weiter bevorzugt 0,05 - 0,5 Gew.-%, außerordentlich bevorzugt 0,1 - 0,2 Gew.-%, Xanthophylle, vorzugsweise Canthaxanthin, Citranaxanthin, Flavoxanthin, Rhodoxanthin, Rubixanthin, Zeaxanthin und ganz besonders bevorzugt Astaxanthin.

Als ein weiteres besonders geeignetes Antioxidans können die erfindungsgemäßen Mittel mindestens einen Stoff aus der Gruppe der Superoxiddismutasen enthalten. Erfindungsgemäß bevorzugte Hautbehandlungsmittel enthalten - bezogen auf ihr Gewicht - 0,001 bis 10 Gew.-%, vorzugsweise 0,005 bis 7,5 Gew.-%, weiter bevorzugt 0,01 bis 5 Gew.-% weiter bevorzugt 0,02 bis 2 Gew.-% und insbesondere 0,1 bis 0,5 Gew.-%, Superoxiddismutase(n).

Unabhängig von der Art des Antioxidans und unabhängig davon, ob ein oder mehrerer Antioxidantien eingesetzt werden, sind erfindungsgemäße Hautbehandlungsmittel bevorzugt, die Antioxidantien - bezogen auf das Gewicht des Hautbehandlungsmittels - in einer Gesamtmenge von 0,00001 - 1 Gew.-%, bevorzugt 0,0001 - 0,6 Gew.-%, besonders bevorzugt 0,001 - 0,5 Gew.-% und außerordentlich bevorzugt 0,01 - 0,3 Gew.-%, enthalten.

Weitere bevorzugte erfindungsgemäße Zusammensetzungen sind dadurch gekennzeichnet, dass zur Verbesserung der Hautfeuchtigkeit unter Verzicht auf komedogene Substanzen mindestens ein alkyl- oder hydroxyalkylsubstituierten Harnstoff der allgemeinen Formel (UREA) enthalten ist, 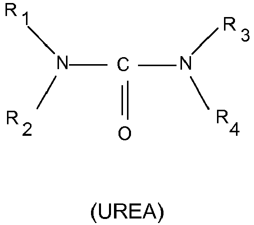 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.

Weitere bevorzugte erfindungsgemäße Zusammensetzungen sind dadurch gekennzeichnet, dass der mindestens eine alkyl- oder hydroxyalkylsubstituierte Harnstoff der allgemeinen Formel (UREA) ausgewählt ist aus Verbindungen, in denen R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-HydroxyalkylGruppen substituiert ist, stehen, mit der Maßgabe, dass mindestens einer der Reste R₁ und R₂ und gleichzeitig mindestens einer der Reste R₃ und R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.

Weitere bevorzugte erfindungsgemäße Zusammensetzungen sind dadurch gekennzeichnet, dass der mindestens eine alkyl- oder hydroxyalkylsubstituierte Harnstoff der allgemeinen Formel (UREA) ausgewählt ist aus Bis-N,N'-(2-hydroxyethyl)harnstoff.

Erfindungsgemäß besonders bevorzugte Zusammensetzungen sind dadurch gekennzeichnet, dass sie mindestens einen alkyl- oder hydroxyalkylsubstituierten Harnstoff gemäß Formel (UREA), insbesondere N,N'-Bis-(2-hydroxyethyl)harnstoff, in einer Gesamtmenge von 0,01 - 50 Gew.-%, bevorzugt 0,1 - 20 Gew.-%, besonders bevorzugt 1-10 Gew.-% und außerordentlich bevorzugt 2-5 Gew.-%, bezogen auf die gesamte Zusammensetzung, enthalten. N,N'-Bis-(2-hydroxyethyl)harnstoff, bei Raumtemperatur ein kristalliner Feststoff, ist beispielsweise als Handelsprodukt Hydrovance der National Starch and Chemical Company als ca. 45 - 55 %ige wässrige Lösung mit einem Gehalt an Harnstoff und Ammoniumlactat erhältlich.

In einer weiteren bevorzugten Ausführungsform enthalten die erfindungsgemäßen Zusammensetzungen mindestens eine natürliche Betainverbindung. Erfindungsgemäße natürliche Betainverbindungen sind natürlich vorkommende Verbindungen mit der Atomgruppierung R₃N⁺-CH₂-X-COO⁻gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl und X = C-C-Einfachbindung (im Falle des Betains) oder X = -CHOH-CH₂- (im Falle des Carnitins).

Erfindungsgemäß besonders bevorzugte Zusammensetzungen sind dadurch gekennzeichnet, dass sie mindestens eine natürliche Betainverbindung in einer Gesamtmenge von 0,05 bis 5 Gew.-%, bevorzugt 0,1 bis 2 Gew.-%, besonders bevorzugt 0,5 bis 1 Gew.-%, jeweils bezogen auf die gesamte Zusammensetzung, enthalten.

In einer weiteren bevorzugten Ausführungsform enthalten die erfindungsgemäßen Zusammensetzungen neben der erfindungsgemäßen Wirkstoffkombination mindestens eine α-Hydroxycarbonsäure, α-Ketocarbonsäure oder β-Hydroxycarbonsäure oder deren Ester-, Lacton- oder Salzform. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methyl-bernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Erfindungsgemäß besonders bevorzugte Zusammensetzungen sind dadurch gekennzeichnet, dass sie neben der erfindungsgemäßen Wirkstoffkombination mindestens eine α-Hydroxycarbonsäure, α-Ketocarbonsäure und/oder β-Hydroxycarbonsäure oder ein Derivat, insbesondere einen Ester, ein Lacton oder ein Salz hiervon, in einer Gesamtmenge von 0,01 - 10 Gew.-%, bevorzugt 0,1-5 Gew.-%, besonders bevorzugt 0,5 - 1 - 2 Gew.-%, jeweils bezogen auf die gesamte Zusammensetzung, enthalten.

In einer weiteren bevorzugten Ausführungsform enthalten die erfindungsgemäßen Zusammensetzungen mindestens einen Extrakt aus Apfelkernextrakten (Pyrus Malus (Apple) Fruit Extract). Derartige Extrakte erhöhen und/oder verbessern die Interaktion zwischen der extrazellulären Matrix und den Fibroblasten. Erfindungsgemäß besonders bevorzugte Apfelkernextrakte sind unter der Handelsbezeichnung Ederline von der Firma Seporga erhältlich. Das Produkt Ederline enthält Phytohormone, Isoflavonoide, Phytosterole, Triterpenoide, Tocopherole und natürliche Wachse. Ederline ist einmal in wasserlöslicher Form als Ederline-H (INCI: PEG-40 Hydrogenated Castor Oil, PPG-2-Ceteareth-9, Pyrus Malus (Apple) Fruit Extract), zum anderen in fettlöslicher Form als Ederline-L (INCI: Hexyldecanol, Pyrus Malus (Apple) Fruit Extract) erhältlich.

Erfindungsgemäß besonders bevorzugte kosmetische oder dermatologische Zusammensetzungen sind dadurch gekennzeichnet, dass sie den Rohstoff Ederline in Mengen von 0,1 - 10 Gew.-%, bevorzugt 1 - 8 Gew.-% und besonders bevorzugt 3-5 Gew.-%, jeweils bezogen auf die gesamte Zusammensetzung, enthalten. Erfindungsgemäß besonders bevorzugte kosmetische oder dermatologische Zusammensetzungen sind dadurch gekennzeichnet, dass sie mindestens einen Apfelkernextrakt in Mengen von 0,00001 - 2 Gew.-%, bevorzugt 0,001 - 1,6 Gew.-% und besonders bevorzugt 0,03 - 1 Gew.-%, jeweils bezogen auf den Gehalt an Aktivsubstanz in der gesamten Zusammensetzung, enthalten.

In einer weiteren bevorzugten Ausführungsform enthalten die erfindungsgemäßen Zusammensetzungen mindestens einen Desoxyzucker oder ein mindestens einen Desoxyzucker-Baustein enthaltendes Polysaccharid.

Erfindungsgemäß bevorzugte Desoxyzucker sind ausgewählt aus Rhamnose und Fucose. Ein erfindungsgemäß besonders bevorzugtes, Desoxyzucker-Bausteine enthaltendes Polysaccharid ist das Handelsprodukt Fucogel® von Solabia mit der INCI-Bezeichnung Biosaccharide Gum-1. Ein weiteres erfindungsgemäß besonders bevorzugtes, Desoxyzucker-Bausteine enthaltendes Polysaccharid ist das Handelsprodukt Rhamnosoft® von Solabia mit der INCI-Bezeichnung Biosaccharide Gum-2. Ein erfindungsgemäß besonders bevorzugtes, Desoxyzucker-Bausteine enthaltendes Polysaccharid ist das Handelsprodukt Fucogenol® von Solabia mit der INCI-Bezeichnung Biosaccharide Gum-3. Ein erfindungsgemäß besonders bevorzugtes, Desoxyzucker-Bausteine enthaltendes Polysaccharid ist das Handelsprodukt Glycofilm® von Solabia mit der INCI-Bezeichnung Biosaccharide Gum-4. Erfindungsgemäß bevorzugt sind weiterhin Mischungen der vorgenannten, mindestens einen Desoxyzucker-Baustein enthaltenden Polysaccharide, beispielsweise die Mischung aus Biosaccharide Gum-2 und Biosaccharide Gum-3, erhältlich als Handelsprodukt Elastinol plus® von Solabia.

Erfindungsgemäß besonders bevorzugte kosmetische oder dermatologische Zusammensetzungen sind dadurch gekennzeichnet, dass sie mindestens einen Desoxyzucker und/ oder mindestens ein Desoxyzucker-Bausteine enthaltendes Polysaccharid in Gesamtmengen von 0,001 bis 5 Gew.-%, bevorzugt 0,01 bis 2 Gew.-% und besonders bevorzugt 0,1 bis 1 Gew.-%, jeweils bezogen auf die gesamte erfindungsgemäße Zusammensetzung, enthalten.

In einer weiteren bevorzugten Ausführungsform enthalten die erfindungsgemäßen Zusammensetzungen mindestens einen organischen hautaufhellenden Wirkstoff. Erfindungsgemäß bevorzugte hautaufhellende Wirkstoffe sind einzeln oder in Mischungen ausgewählt aus Hydrochinon, Kojisäure, Arbutin, Extrakten aus Süßholzwurzel (Glycyrrhiza glabra), Extrakten aus verschiedenen Teilen des Maulbeerbaums (Morus spp., insbesondere aus Morus alba und hier insbesondere Extrakte aus der Baumrinde, dem Stamm, den Blättern und den Früchten), Extrakten aus Scutellaria spp. (Helmkraut), insbesondere aus Scutellaria baicalensis (Baikal-Helmkraut) und hier insbesondere Extrakte aus der Wurzel, Extrakten aus *Waltheria indica* ("Sleepy Morning") und hier insbesondere Extrakte aus den Blättern, Extrakten aus Bärentraube (*Arctostaphylos uva-ursi* (L.), Ericaceae) und hier insbesondere Extrakte aus den Blättern, Extrakten aus Preiselbeere (Blätter und Blüten), Extrakten aus Heidelbeere (Früchte), Extrakten aus Blattknospen von Birnbäumen, Anissamenöl, Extrakten aus Brombeerblättern, weiterhin Extrakten aus *Pyrola rotundifolia* (Rundblättriges Wintergrün), Gurke und/oder Limone, weiterhin Ascorbinsäure, Cumarinsäure ((Z)-2-Hydroxyzimtsäure) und cis-9-Octadecendisäure (andere Nomenklatur: cis-8-Hexa-decendicarbonsäure), letztere kommerziell erhältlich z. B. unter der Bezeichnung Arlatone DIOIC DCA von Uniqema, und den Estern und/oder Salzen dieser Säuren. Erfindungsgemäß besonders bevorzugte kosmetische oder dermatologische Zusammensetzungen sind dadurch gekennzeichnet, dass sie mindestens einen organischen hautaufhellenden Wirkstoff in Gesamtmengen von 0,00001 bis 10 Gew.-%, bevorzugt 0,01 bis 5 Gew.-% und besonders bevorzugt 0,1 bis 1 -2 Gew.-%, jeweils bezogen auf Aktivsubstanz in der gesamten erfindungsgemäßen Zusammensetzung, enthalten.

Vorteilhafterweise liegen die erfindungsgemäßen Hautbehandlungsmittel in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer besonderen Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die so genannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

Ein weiterer Gegenstand der vorliegenden Erfindung ist die Verwendung von erfindungsgemäßen Hautbehandlungsmitteln zur nicht-therapeutischen, kosmetischen Behandlung und/oder Minimierung von Hautfalten und -fältchen, den Anzeichen der intrinsischen und extrinsischen Hautalterung, müder und/oder schlaffer Haut und/oder UV-geschädigter Haut.

Bezüglich weiterer bevorzugter Ausführungsformen der erfindungsgemäßen Verwendungen gilt mutatis mutandis das zu den erfindungsgemäßen Mitteln Gesagte.

Ein weiterer Gegenstand der vorliegenden Erfindung ist ein nicht-therapeutisches Verfahren zur nicht-therapeutischen, kosmetischen Behandlung und/oder Minimierung von Hautfalten und -fältchen, den Anzeichen der intrinsischen und extrinsischen Hautalterung, müder und/oder schlaffer Haut und/oder UV-geschädigter Haut, dadurch gekennzeichnet, dass ein erfindungsgemäßes Hautbehandlungsmittel auf die Haut aufgetragen wird.

Auch bezüglich weiterer bevorzugter Ausführungsformen der erfindungsgemäßen Verfahren gilt mutatis mutandis das zu den erfindungsgemäßen Mitteln Gesagte.

Die folgenden Beispiele sollen den Gegenstand der vorliegenden Anmeldung verdeutlichen, ohne ihn hierauf zu beschränken. Alle Mengenangaben in den folgenden Tabellen sind in Gew.-%.

**Tabelle 1: Öl-in-Wasser-Emulsionen**

| | 1 | 2 | 3 | 4 | 5 |
|---|---|---|---|---|---|
| Distelöl | 3,00 | 3,00 | 3,00 | 3,00 | 3,00 |
| Caprylic/Capric Triglyceride | 5,00 | 5,00 | 5,00 | 5,00 | 5,00 |
| Novata AB | 2,00 | 2,00 | 2,00 | 2,00 | 2,00 |
| Lanette 22 | 1,00 | 1,00 | 1,00 | 1,00 | 1,00 |
| Cutina MD | 2,00 | 2,00 | 2,00 | 2,00 | 2,00 |
| Stenol 1618 | 1,00 | 1,00 | 1,00 | 1,00 | 1,00 |
| Baysilon M350 | 1,00 | 1,00 | 1,00 | 1,00 | 1,00 |
| Propylparaben | 0,20 | 0,20 | 0,20 | 0,20 | 0,20 |
| Dry Flo Plus | 1,00 | 2,00 | 3,00 | 2,00 | 3,00 |
| Propylenglycol | 5,00 | 5,00 | 5,00 | 5,00 | 5,00 |
| Glycerin | 5,00 | 3,00 | 3,00 | 3,00 | 3,00 |
| Methylparaben | 0,20 | 0,20 | 0,20 | 0,20 | 0,20 |
| Phenoxyethanol | 0,9 | 0,5 | 0,9 | 0,5 | 0,9 |
| Parfum | 0,10 | 0,10 | 0,10 | 0,10 | 0,10 |
| Benzylidene Dimethoxydimethylindanone | 0,1 | 0,2 | 0,05 | 0,3 | 0,05 |
| Photosomes | 0,1 | 1,0 | 1,0 | 0,5 | 0,5 |
| Astaxanthin | - | - | - | - | 0,05 |
| 3,4-Dihydro-7-methoxy-2,2-dimethyl-2H-1-benzopyran-6-ol | - | 0,01 | - | - | - |
| Dihydroquercetin | - | - | 0,5 | - | - |
| pulverisierter Traubenkernextrakt (Vitis vinifera seed), 95 Gew.-% Polyphenole | - | - | - | 0,5 | - |
| Wasser | ad 100 | ad 100 | ad 100 | ad 100 | ad 100 |

**Tabelle 2: Öl-in-Wasser-Emulsionen**

| | **1** | **2** | **3** | **4** | **5** |
|---|---|---|---|---|---|
| Gehärtetes Sojalecithin | 0,50 | 0,50 | 0,50 | 0,50 | 0,50 |
| Cutina MD | 1,00 | 1,00 | 1,00 | 1,00 | 1,00 |
| Lanette 22 | 2,00 | 2,00 | 2,00 | 2,00 | 2,00 |
| Baysilone M 350 | 0,50 | 0,50 | 0,50 | 0,50 | 0,50 |
| Propylparaben | 0,20 | 0,20 | 0,20 | 0,20 | 0,20 |
| Dow Corning 9040 | 1,00 | 1,00 | 1,00 | 1,00 | 1,00 |
| Glycerin | 4,50 | 3,00 | 3,00 | 3,00 | 4,50 |
| Hexandiol | 6,00 | 3,00 | | 3,00 | 6,00 |
| Methylparaben | 0,20 | 0,20 | 0,20 | 0,20 | 0,20 |
| Tego Carbomer 140 | 0,30 | 0,30 | 0,30 | 0,30 | 0,30 |
| DSH C N | 5,00 | 5,00 | 5,00 | 5,00 | 5,00 |
| Propylenglycol | 5,00 | 5,00 | 5,00 | 5,00 | 5,00 |
| Simulgel EPG | 1,50 | 1,50 | 1,50 | 1,50 | 1,50 |
| TiO₂ (Füllstoff) | 0,50 | 0,50 | 0,50 | 0,50 | 0,50 |
| Benzylidene Dimethoxydimethylindanone | 0,3 | 0,2 | 0,1 | 0,08 | 0,2 |
| Photosomes | 1,0 | 2,0 | 2,0 | 0,5 | 0,1 |
| Zeaxanthin | 0,1 | - | - | - | - |
| 3,4-Dihydro-7-methoxy-2,2-dimethyl-2H-1-benzopyran-6-ol | - | 0,01 | - | - | - |
| Lycopin | - | - | - | - | 0,07 |
| Wasser | ad 100 | ad 100 | ad 100 | ad 100 | ad 100 |

**Tabelle 3: Öl-in-Wasser-Emulsionenmit Sonnenschutzfaktor**

| | 1 | 2 | 3 | 4 | 5 |
|---|---|---|---|---|---|
| Emulsiphos 677660 | 3 | 3 | 3 | 3 | 3 |
| Dibutyladipat | 5 | 5 | 5 | 5 | 5 |
| Caprylic/Capric Triglyceride | 3,5 | 3,5 | 3,5 | 3,5 | 3,5 |
| Safloröl raffiniert | 2 | 2 | 2 | 2 | 2 |
| Behenylalkohol | 3 | 3 | 3 | 3 | 3 |
| 2-Propylheptyl octanoate | 1 | 1 | 1 | 1 | 1 |
| Bienenwachs | 0,2 | 0,2 | 0,2 | 0,2 | 0,2 |
| COSMEDIA SP | 0,5 | 0,5 | 0,5 | 0,5 | 0,5 |
| Butylmethoxydibenzoylmethan | 3 | 3 | 3 | 3 | 3 |
| Parsol SLX | 3 | 3 | 3 | 3 | 3 |
| Propylparaben | 0,2 | 0,2 | 0,2 | 0,2 | 0,2 |
| Hexandiol-1,6 | 5 | 5 | 5 | 5 | 5 |
| Glycerin 86% | 4,5 | 4,5 | 4,5 | 4,5 | 4,5 |
| Sorbit 70% | 2 | 2 | 2 | 2 | 2 |
| Methylparaben | 0,2 | 0,2 | 0,2 | 0,2 | 0,2 |
| Tego Carbomer 140 | 0,5 | 0,5 | 0,5 | 0,5 | 0,5 |
| Trinatriumnitrilotriacetat | 0,04 | 0,04 | 0,04 | 0,04 | 0,04 |
| Cosmedia Silc | 2 | 2 | 2 | 2 | 2 |
| Aminomethyl propanol (99%) | 0,99 | 0,99 | 0,99 | 0,99 | 0,99 |
| Neo Heliopan Hydro | 3 | 3 | 3 | 3 | 3 |
| Natriumhydroxid Perlen | 0,365 | 0,365 | 0,365 | 0,365 | 0,365 |
| Benzylidene Dimethoxydimethylindanone | 0,1 | 0,1 | 0,2 | 0,2 | 0,1 |
| Photosomes | 3,0 | 2,0 | 2,0 | 3,0 | 1,0 |
| Wasser, vollentsalzt | ad 100 | ad 100 | ad 100 | ad 100 | ad 100 |

**Tabelle 4: Öl-in-Wasser-Emulsionen**

| | **1** | **2** | **3** | **4** | **5** |
|---|---|---|---|---|---|
| Montanov 202 | 5,0 | 5,0 | 5,0 | 5,0 | 5,0 |
| Cutina MD | 2,0 | 2,0 | 2,0 | 2,0 | 2,0 |
| Cetearyl Alcohol | 1,0 | 1,0 | 1,0 | 1,0 | 1,0 |
| Dibutyladipat | 9,0 | 9,0 | 9,0 | 9,0 | 9,0 |
| Dow Corning 245 | - | 7,0 | 3,0 | - | 7,0 |
| Dow Corning 9040 | 1,0 | 1,0 | - | 1,0 | 1,0 |
| Parfum | 0,3 | 0,3 | 0,3 | 0,3 | 0,3 |
| Fucogel 1000 | - | 2,0 | - | - | 2,0 |
| Keltrol SF | - | 0,2 | 0,2 | - | 0,2 |
| Aristoflex AVC | - | - | 0,5 | - | - |
| Hexandiol-1,6 | 6,0 | 6,0 | - | 6,0 | 6,0 |
| Glycerin | 3,0 | 5,0 | 10,0 | 3,0 | 5,0 |
| Dow Corning 200 Fluid (5 cSt) | 7,0 | 7,0 | - | 7,0 | 7,0 |
| Dow Corning 245 | - | 5,0 | 3,0 | - | 5,0 |
| Dow Corning 9040 | 1,0 | - | 1,0 | 1,0 | - |
| Propylenglycol | 2,0 | 2,0 | - | 2,0 | 2,0 |
| Tego Carbomer 140 | 0,3 | 0,3 | 0,3 | 0,3 | 0,3 |
| Dry Flo Plus | 1,0 | 1,0 | 1,0 | 1,0 | 1,0 |
| Benzylidene Dimethoxydimethylindanone | 0,3 | 0,4 | 0,5 | 0,1 | 0,1 |
| Ultrasomes | 0,1 | 0,5 | 1,0 | 2,0 | 0,5 |
| Photosomes | - | - | - | 1,0 | 0,5 |
| Astaxanthin | 0,1 | - | - | - | - |
| Lutein | - | - | 0,03 | | - |
| Coenzym Q-10 | - | - | - | - | 0,05 |
| Wasser | ad 100 | ad 100 | ad 100 | ad 100 | ad 100 |

**Liste der verwendeten Rohstoffe**

| Handelsname | INCI-Bezeichnung | Lieferant/Herstel ler |
|---|---|---|
| Aristoflex AVC | Ammonium Acryloyldimethyl Taurate/VP Copolymer/, t-Butyl Alcohol | Clariant |
| Baysilon M 350 | Dimethicone (350 cSt) | GE Bayer Silicones |
| Cosmedia Silc | Silica | Cognis |
| Cosmedia SP | Sodium Polyacrylate | Cognis |
| Cutina MD | Glyceryl Stearate (Mischung aus Glycerylmono- und distearat) | Cognis |
| Dow Corning 9040 | Cyclomethicone/ Dimethicone Crosspolymer (87-88 Gew.-%/12-13 Gew.-%) | Dow Corning |
| Dow Corning®200 Fluid, 5 cSt | Dimethicone | Dow Corning |
| Dow Corning®245 Fluid | Cyclomethicone | Dow Corning |
| Dry Flo Plus | Aluminium Starch Octenylsuccinate | National Starch |
| DSH C N | Water, Dimethylsilanol Hyaluronate | Exsymol |
| Emulsiphos 677660 | Potassium Cetyl Phosphate, Hydrogenated Palm Glycerides | Symrise |
| Fucogel 1000 | Aqua, Biosaccharide Gum-1 (1,1 Gew.-%) | Solabia |
| Keltrol SF | Xanthan Gum | Kelco |
| Lanette® 22 | Behenyl Alcohol | Cognis |
| Montanov 202 | Arachidyl Alkohol, Behenyl Alcohol, Arachidyl Glucoside | Seppic |
| Neo Heliopan Hydro | Phenylbenzimidazol sulfonic acid | Symrise |
| Novata AB | Cocoglycerides | Cognis |
| Parsol SLX | Polysilicone-15 | DSM |
| Photosomes | Aqua, Lecithine, Plancton Extract | AGI Dermatics |
| Simulgel EPG | Aqua (Water)/Sodium Acrylate / Sodium Acryloyldimethyl Taurate Copolymer / Polyisobutene / Caprylic/Capryl Glucoside | Seppic |
| Stenol 16/18 | Cetearyl alcohol | Cognis |
| SymHelios 1031 | Benzylidene Dimethoxydimethylindanone (CAS 924626-15-3), 2,3-Dihydro-5,6-dimethoxy-3,3-dimethyl-2-(phenylmethylene)-1H-Inden-1-one | Symrise |
| Tego Carbomer 140 | Carbomer | Degussa |
| Ultrasomes | Aqua, Lecithine, Micrococcus Luteus Extract | AGI Dermatics |

## Patentansprüche

1. Kosmetisches topisches Hautbehandlungsmittel, enthaltend in einem geeigneten Träger
a. mindestens eine Verbindung der Formel (I) 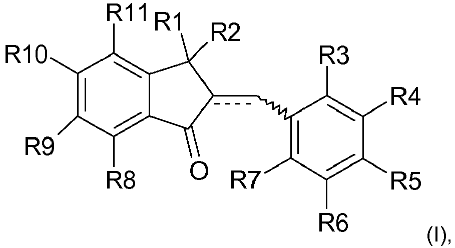 in der
- die Reste R1 und R2 unabhängig voneinander für -H oder -CH₃ oder -CH₂CH₃ oder -CH(CH₃)₂ oder -(CH₂)ₙCH₃ mit n = 2 bis 11 stehen,
- die Reste R3 bis R11 unabhängig voneinander für -H oder -CH₃ oder -CH₂CH₃ oder -CH(CH₃)₂ oder -(CH₂)ₖCH₃ mit k = 2 bis 11 oder -OH oder -OCH₃ oder -OCH₂CH₃ oder -OCH(CH₃)₂ oder -O(CH₂)ₘCH₃ mit m = 2 bis 11 stehen,
- die gestrichelte Linie für eine Doppelbindung oder 2 Wasserstoffatome steht,
b. mindestens ein DNA-Reparaturenzym.

2. Hautbehandlungsmittel nach Anspruch 1, **dadurch gekennzeichnet, dass** es mindestens eine Verbindung der Formel (I) enthält, in der die Reste R1 und R2 für eine Methylgruppe stehen.

3. Hautbehandlungsmittel nach einem der Ansprüche 1 oder 2, **dadurch gekennzeichnet, dass** es mindestens eine Verbindung der Formel (I) enthält, in der die Reste R4, R5, R9 und R10 unabhängig voneinander ausgewählt sind aus -H oder -OCH₃, wobei Verbindungen bevorzugt sind, die insgesamt eine oder zwei -OCH₃ Gruppen im Molekül enthalten.

4. Hautbehandlungsmittel nach einem der Ansprüche 1 bis 3, **dadurch gekennzeichnet, dass** es mindestens eine Verbindung der Formel (I) enthält, in der der Rest R9 eine Methylgruppe ist.

5. Hautbehandlungsmittel nach einem der Ansprüche 1 bis 4, **dadurch gekennzeichnet, dass** es mindestens eine Verbindung der Formel (I) enthält, in der der Rest R10 eine -O(CH₂)₃CH₃ Gruppe ist.

6. Hautbehandlungsmittel nach einem der Ansprüche 1 bis 5, enthaltend mindestens eine Verbindung der Formeln (Ia) bis (Ih):

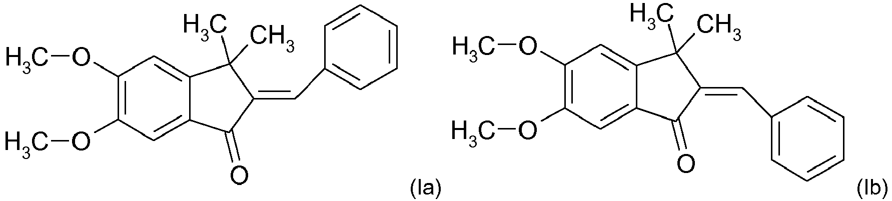

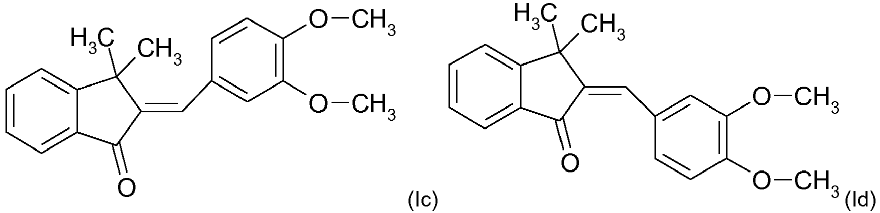

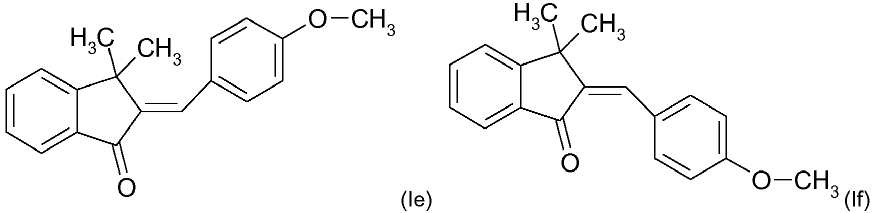

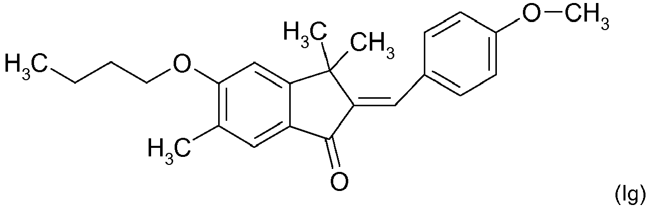

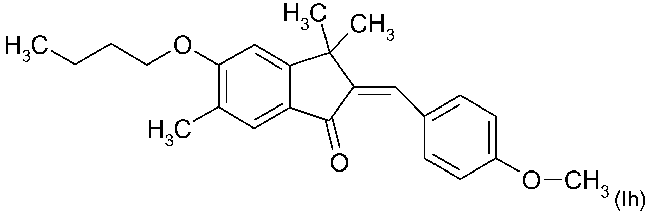

7. Hautbehandlungsmittel nach einem der Ansprüche 1 bis 6, **dadurch gekennzeichnet, dass** es die Verbindung(en) der Formel (I) - bezogen auf das Gewicht des Hautbehandlungsmittels - in Mengen von 0,001 bis 10 Gew.-%, vorzugsweise von 0,005 bis 7,5 Gew.-%, weiter bevorzugt von 0,01 bis 5 Gew.-%, noch weiter bevorzugt von 0,025 bis 4 und insbesondere von 0,05 bis 3 Gew.-% enthält.

8. Hautbehandlungsmittel nach einem der Ansprüche 1 bis 7, **dadurch gekennzeichnet, dass** es als DNA-Reparatur-Enzym Photolyase enthält.

9. Hautbehandlungsmittel nach einem der Ansprüche 1 bis 8, **dadurch gekennzeichnet, dass** es als DNA-Reparatur-Enzym T4 Endonuclease V enthält.

10. Hautbehandlungsmittel nach einem der Ansprüche 1 bis 9, **dadurch gekennzeichnet, dass** es als DNA-Reparatur-Enzym 8-Oxoguanin-glycosylase enthält.

11. Hautbehandlungsmittel nach einem der Ansprüche 1 bis 10, **dadurch gekennzeichnet, dass** es das/die DNA-Reparaturenzym(e) - bezogen auf das Gewicht des Hautbehandlungsmittels - in Mengen von 0,00001 - 1 Gew.-%, bevorzugt 0,0001 - 0,1 Gew.-%, besonders bevorzugt 0,001 -0,05 Gew.-% und außerordentlich bevorzugt 0,002 - 0,005 Gew.-%, enthält.

12. Hautbehandlungsmittel nach einem der Ansprüche 1 bis 11, **dadurch gekennzeichnet, dass** es zusätzlich mindestens ein Antioxidationsmittel aus der Gruppe
- der 6,7-disubstituierten 2,2-Dialkylchromanen oder -chromenen der allgemeinen Formeln (II) oder (III), 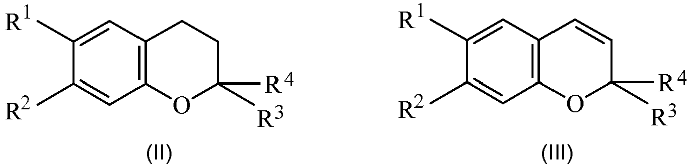 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen, insbesondere Lipochroman-6 und/oder
- der Ubichinone, insbesondere Coenzym Q10, und/oder
- der Quercetine und/oder
- Ellagsäure und/oder Ellagate und/oder
- der Hydroxystilben-Oligomeren, deren Alkylethern und/oder deren Estern und/oder
- der Xanthophylle, insbesondere Astaxanthin, und/oder
- der Superoxiddismutasen
enthält.

13. Hautbehandlungsmittel nach Anspruch 12, **dadurch gekennzeichnet, dass** es die Antioxidantien - bezogen auf das Gewicht des Hautbehandlungsmittels - in einer Gesamtmenge von 0,00001 - 1 Gew.-%, bevorzugt 0,0001 - 0,6 Gew.-%, besonders bevorzugt 0,001 - 0,5 Gew.-% und außerordentlich bevorzugt 0,01 - 0,3 Gew.-%, enthält.

14. Verwendung eines Hautbehandlungsmittels nach einem der Ansprüche 1 bis 13 zur nicht-therapeutischen, kosmetischen Behandlung und/oder Minimierung von Hautfalten und - fältchen, den Anzeichen der intrinsischen und extrinsischen Hautalterung, müder und/oder schlaffer Haut und/oder UV-geschädigter Haut.

15. Nicht-therapeutisches Verfahren zur nicht-therapeutischen, kosmetischen Behandlung und/oder Minimierung von Hautfalten und -fältchen, den Anzeichen der intrinsischen und extrinsischen Hautalterung, müder und/oder schlaffer Haut und/oder UV-geschädigter Haut, **dadurch gekennzeichnet, dass** ein Hautbehandlungsmittel gemäß einem der Ansprüche 1 bis 13 auf die Haut aufgetragen wird.

## Claims

1. A cosmetic topical skin treatment agent containing, in a suitable carrier,
a. at least one compound of formula (I) 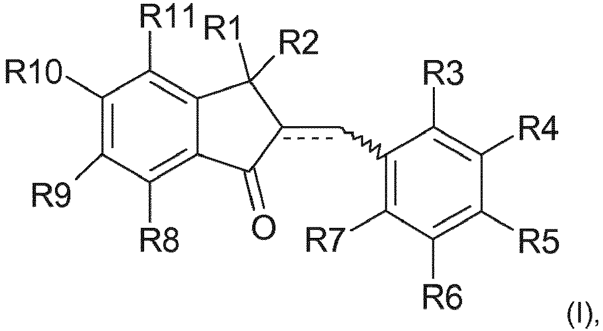 in which
- the functional groups R1 and R2 represent, independently of one another, -H or -CH₃ or -CH₂CH₃ or -CH(CH₃)₂ or -(CH₂)ₙCH₃, where n = 2 to 11,
- the functional groups R3 to R11 represent, independently of one another, -H or -CH₃ or -CH₂CH₃ or -CH(CH₃)₂ or -(CH₂)ₖCH₃, where k = 2 to 11, or -OH or -OCH₃ or -OCH₂CH₃ or -OCH(CH₃)₂ or
- O(CH₂)ₘCH₃, where m = 2 to 11,
- the dashed line represents a double bond or two hydrogen atoms,
b. at least one DNA repair enzyme.

2. The skin treatment agent according to claim 1, **characterized in that** it contains at least one compound of formula (I) in which the functional groups R1 and R2 represent a methyl group.

3. The skin treatment agent according to one of claims 1 or 2, **characterized in that** it contains at least one compound of formula (I) in which the functional groups R4, R5, R9 and R10 are selected, independently of one another, from -H or -OCH₃, compounds that contain a total of one or two - OCH₃ groups in the molecule being preferred.

4. The skin treatment agent according to one of claims 1 to 3, **characterized in that** it contains at least one compound of formula (I) in which the functional group R9 is a methyl group.

5. The skin treatment agent according to one of claims 1 to 4, **characterized in that** it contains at least one compound of formula (I) in which the functional group R10 is an -O(CH₂)₃CH₃ group.

6. The skin treatment agent according to one of claims 1 to 5, containing at least one compound of formulas (Ia) to (Ih):

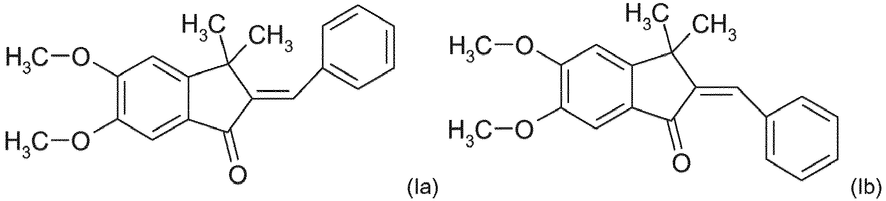

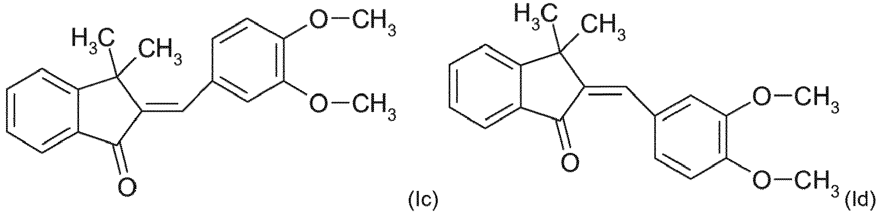

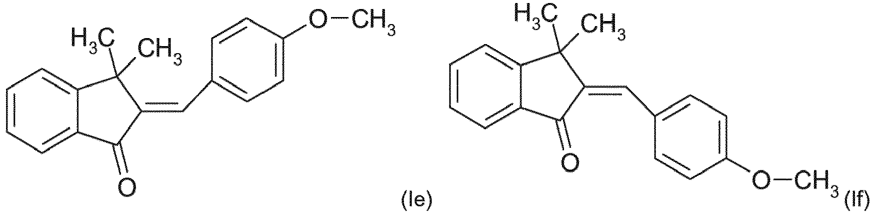

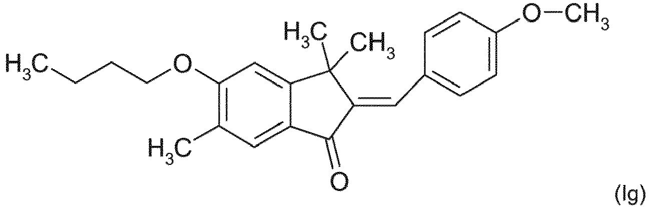

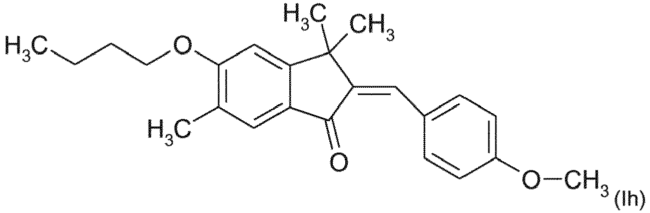

7. The skin treatment agent according to one of claims 1 to 6, **characterized in that** it contains the compound(s) of formula (I) in amounts of from 0.001 to 10 wt.%, preferably from 0.005 to 7.5 wt.%, more preferably from 0.01 to 5 wt.%, even more preferably from 0.025 to 4 wt.%, and in particular from 0.05 to 3 wt.%, based on the weight of the skin treatment agent.

8. The skin treatment agent according to one of claims 1 to 7, **characterized in that** it contains photolyase as the DNA repair enzyme.

9. The skin treatment agent according to one of claims 1 to 8, **characterized in that** it contains T4 endonuclease V as the DNA repair enzyme.

10. The skin treatment agent according to one of claims 1 to 9, **characterized in that** it contains 8-oxoguanine glycosylase as the DNA repair enzyme.

11. The skin treatment agent according to one of claims 1 to 10, **characterized in that** it contains the DNA repair enzyme(s) in amounts of from 0.00001 to 1 wt.%, preferably from 0.0001 to 0.1 wt.%, particularly preferably from 0.001 to 0.05 wt.%, and extremely preferably from 0.002 to 0.005 wt.%, based on the weight of the skin treatment agent.

12. The skin treatment agent according to one of claims 1 to 11, **characterized in that** it further contains at least one antioxidant from the group of
- 6,7-disubstituted 2,2-dialkyl chromanes or chromenes of general formulas (II) or (III), 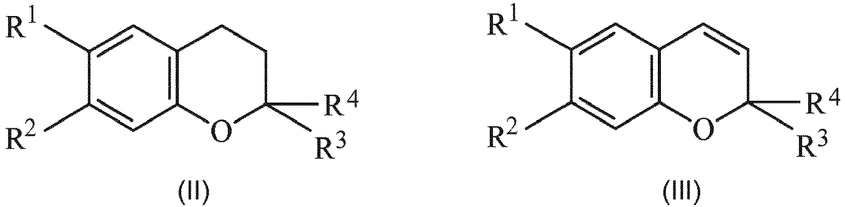 where R¹ and R² are, independently of one another, an OH group, a methoxy group or a CF₃CH₂O group, and R³ and R⁴ are, independently of one another, a C₁-C₄ alkyl group, in particular Lipochroman-6 and/or
- ubiquinone, in particular Coenzyme Q10, and/or
- quercetin and/or
- ellagic acid and/or ellagate and/or
- hydroxystilbene oligomers, the alkyl ethers thereof and/or the esters thereof and/or
- xanthophylls, in particular astaxanthin, and/or
- superoxide dismutases.

13. The skin treatment agent according to claim 12, **characterized in that** it contains the antioxidants in a total amount of from 0.00001 to 1 wt.%, preferably from 0.0001 to 0.6 wt.%, particularly preferably from 0.001 to 0.5 wt.%, and extremely preferably from 0.01 to 0.3 wt.%, based on the weight of the skin treatment agent.

14. The use of a skin treatment agent according to one of claims 1 to 13 for non-therapeutically and cosmetically treating and/or minimizing wrinkles and fine lines, signs of intrinsic and extrinsic skin aging, tired and/or loose skin and/or UV-damaged skin.

15. A non-therapeutic method for non-therapeutically and cosmetically treating and/or minimizing wrinkles and fine lines, signs of intrinsic and extrinsic skin aging, tired and/or loose skin and/or UV-damaged skin, **characterized in that** a skin treatment agent according to one of claims 1 to 13 is applied to the skin.

## Revendications

1. Agent de traitement de la peau cosmétique topique, contenant dans un support adapté
a. au moins un composé de formule (I) 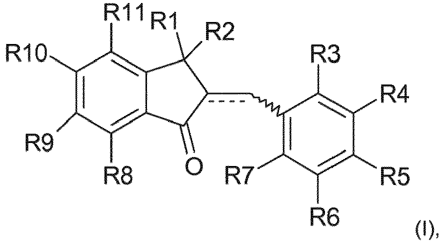 dans laquelle
- les radicaux R1 et R2 représentent indépendamment l'un de l'autre -H ou -CH₃ ou -CH₂CH₃ ou -CH(CH₃)₂ ou -(CH₂)ₙCH₃, où n = 2 à 11,
- les radicaux R3 à R11 représentent indépendamment les uns des autres -H ou -CH₃ ou - CH₂CH₃ ou -CH(CH₃)₂ ou -(CH₂)ₖCH₃, où k = 2 à 11, ou -OH ou -OCH₃ ou -OCH₂CH₃ ou -OCH(CH₃)₂ ou -O(CH₂)ₘCH₃ où m = 2 à 11,
- la ligne en pointillés représente une liaison double ou 2 atomes d'hydrogène,
b. au moins une enzyme de réparation de l'ADN.

2. Agent de traitement de la peau selon la revendication 1, **caractérisé en ce qu'**il contient au moins un composé de formule (I), dans laquelle les radicaux R1 et R2 représentent un groupe méthyle.

3. Agent de traitement de la peau selon l'une des revendications 1 ou 2, **caractérisé en ce qu'**il contient au moins un composé de formule (I), dans laquelle les radicaux R4, R5, R9 et R10 sont choisis indépendamment les uns des autres parmi -H ou -OCH₃, les composés contenant au total un ou deux groupes -OCH₃ dans la molécule étant préférés.

4. Agent de traitement de la peau selon l'une des revendications 1 à 3, **caractérisé en ce qu'**il contient au moins un composé de formule (I), dans laquelle le radical R9 est un groupe méthyle.

5. Agent de traitement de la peau selon l'une des revendications 1 à 4, **caractérisé en ce qu'**il contient au moins un composé de formule (I), dans laquelle le radical R10 est un groupe - O(CH₂)₃CH₃.

6. Agent de traitement de la peau selon l'un des revendications 1 à 5, contenant au moins un composé selon les formules (Ia) à (Ih) :

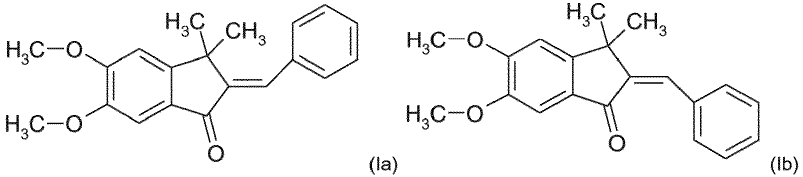

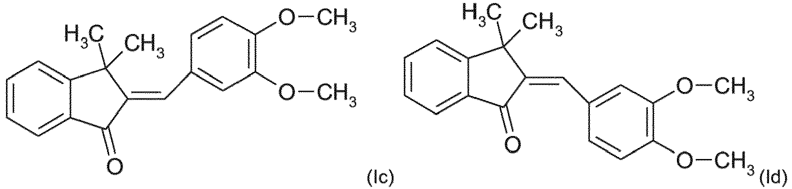

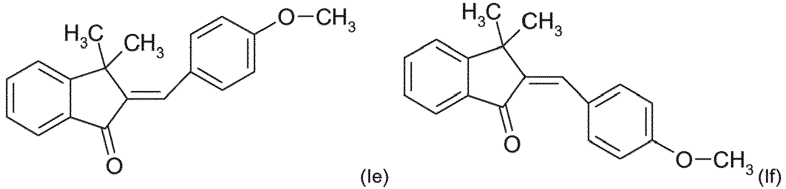

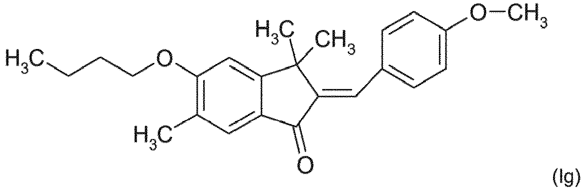

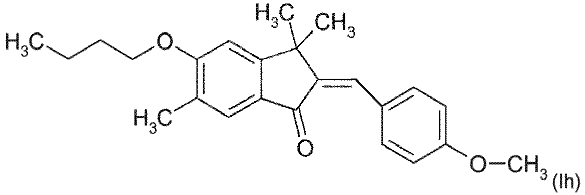

7. Agent de traitement de la peau selon l'une des revendications 1 à 6, **caractérisé en ce qu'**il contient le ou les composé(s) de formule (I), par rapport au poids de l'agent de traitement de la peau, dans des quantités de 0,001 à 10 % en poids, de préférence de 0,005 à 7,5 % en poids, de manière davantage préférée de 0,01 à 5 % en poids, de manière encore davantage préférée de 0,025 à 4 et en particulier de 0,05 à 3 % en poids.

8. Agent de traitement de la peau selon l'une des revendications 1 à 7, **caractérisé en ce que** qu'il contient comme enzyme de réparation de l'ADN de la photolyase.

9. Agent de traitement de la peau selon l'une des revendications 1 à 8, **caractérisé en ce qu'**il contient comme enzyme de réparation de l'ADN de la T4 endonucléase V.

10. Agent de traitement de la peau selon l'une des revendications 1 à 9, **caractérisé en ce qu'**il contient comme enzyme de réparation de l'ADN de la 8-oxoguanine-glycosylase.

11. Agent de traitement de la peau selon l'une des revendications 1 à 10, **caractérisé en ce qu'**il contient la ou les enzymes de réparation de l'ADN, par rapport au poids de l'agent de traitement de la peau, dans des quantités de 0,00001 à 1 % en poids, de préférence de 0,0001 à 0,1 % en poids, de manière particulièrement préférée de 0,001 à 0,05 % en poids et de manière préférée entre toutes de 0,002 à 0,005 % en poids.

12. Agent de traitement de la peau selon l'une des revendications 1 à 11, **caractérisé en ce qu'**il contient en plus au moins un agent antioxydant issu du groupe
- des 2,2-dialkylchromanes ou chromènes disubstitués en 6,7 de formules générales (II) ou (III), 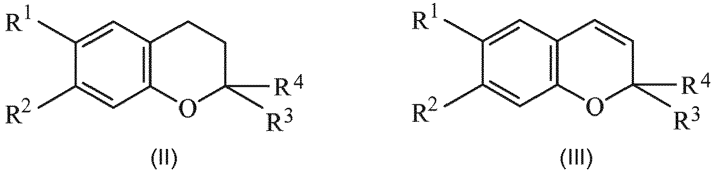 dans lesquelles R¹ et R² représentent indépendamment l'un de l'autre un groupe OH, un groupe méthoxy ou un groupe CF₃CH₂O et R³ et R⁴ représentent indépendamment l'un de l'autre un groupe alkyle en C₁ à C₄, en particulier le lipochromane-6 et/ou
- l'ubiquinone, en particulier la coenzyme Q10, et/ou
- la quercétine et/ou
- l'acide ellagique et/ou des ellagates et/ou
- des oligomères de l'hydroxystilbène, ses alkyléthers et/ou ses esters et/ou
- la xantophylle, en particulier l'astaxanthine, et/ou
- les superoxydes dismutases.

13. Agent de traitement de la peau selon la revendication 12, **caractérisé en ce qu'**il contient les antioxydants, par rapport au poids de l'agent de traitement de la peau, dans une quantité totale de 0,00001 à 1 % en poids, de préférence de 0,0001 à 0,6 % en poids, de manière particulièrement préférée de 0,001 à 0,5 % en poids et de manière préférée entre toutes de 0,01 à 0,3 % en poids.

14. Utilisation d'un agent de traitement de la peau selon l'une des revendications 1 à 13, pour le traitement cosmétique, non thérapeutique et/ou la minimisation de rides et ridules cutanées, des signes du vieillissement intrinsèque et extrinsèque de la peau, d'une peau fatiguée et/ou relâchée et/ou abîmée par les UV.

15. Procédé non thérapeutique destiné au traitement cosmétique, non-thérapeutique et/ou à la minimisation de rides et ridules cutanées, des signes du vieillissement intrinsèque et extrinsèque de la peau, d'une peau fatiguée et/ou relâchée et/ou abîmée par les UV, **caractérisé en ce qu'**un agent de traitement de la peau selon l'une des revendications 1 à 13 est appliqué sur la peau.
